# EUROPEAN PATENT SPECIFICATION

(11) **EP 3 025 716 B1**
(45) Date of publication and mention of the grant of the patent: **04.04.2018**
(21) Application number: 13749318.5
(22) Date of filing: 07.02.2013
(51) Int. Cl.: A61K 31/50, A61K 31/5025, A61K 31/53, A61K 31/551, A61K 31/5517, G01N 33/94, A61P 25/02, A61P 29/00

(54) **SUBSTANCE FOR TREATMENT OR RELIEF OF PAIN**
SUBSTANZ ZUR BEHANDLUNG ODER LINDERUNG VON SCHMERZEN
SUBSTANCE POUR LE TRAITEMENT OU LE SOULAGEMENT DE LA DOULEUR

(30) Priority: 14.02.2012 CN 201210032922; 30.11.2012 CN 201210506153
(43) Date of publication of application: 01.06.2016
(73) Proprietor: Shanghai Institutes for Biological Sciences, Chinese Academy of Sciences, Shanghai 200031 (CN); Shanghai Institute Materia Medica, Chinese Academy Of Sciences, Pudong, Shanghai 201203 (CN)
(72) Inventor: ZHANG, Xu, Shanghai 200031 (CN); LI, Shuai, Shanghai 200031 (CN); BAO, Lan, Shanghai 200031 (CN); YE, Yang, Shanghai 201203 (CN); YAO, Sheng, Shanghai 201203 (CN)
(74) Representative: Dupuis-Latour, Dominique
(86) International application number: PCT/CN2013/071515
(87) International publication number: WO 2013/120438

(56) References cited:
- WO-A1-00/23449
- WO-A1-03/099816
- WO-A1-03/099816
- WO-A1-2011/020044
- WO-A1-2011/024115
- WO-A2-01/38331
- Hans Ulrich Zeilhofer ET AL: "Chronic Pain States: Pharmacological Strategies to Restore Diminished Inhibitory Spinal Pain Control", Annual Review of Pharmacology and Toxicology, 15 August 2011 (2011-08-15), pages 111-133, XP055271498, Retrieved from the Internet: URL:http://www.annualreviews.org/doi/full/ 10.1146/annurev-pharmtox-010611-134636 [retrieved on 2016-05-10]
- Naheed R Mirza ET AL: "The role of GABA(A) receptor subtypes as analgesic targets", Drug news & perspectives, 1 July 2010 (2010-07-01), page 351, XP055271563, United States DOI: 10.1358/dnp.2010.23.6.1489909 Retrieved from the Internet: URL:https://www.researchgate.net/profile/G ordon_Munro2/publication/45584035_The_role _of_GABAA_receptor_subtypes_as_analgesic_t argets/links/56262ad808aed3d3f137e937.pdf [retrieved on 2016-05-10]
- BRAVO-HERNÁNDEZ MARIANA ET AL: "Evidence for the participation of peripheral [alpha]5subunit-containing GABAAreceptors in GABAAagonists-induced nociception in", EUROPEAN JOURNAL OF PHARMACOLOGY, vol. 734, 12 April 2014 (2014-04-12), pages 91-97, XP029028866, ISSN: 0014-2999, DOI: 10.1016/J.EJPHAR.2014.03.051
- ALESSANDRA DI LIO ET AL.: 'HZ166, a Novel GABAA Receptor Subtype-selective Benzodiazepine Site Ligand, is Antihyperalgesic in Mouse Models of Inflammatory And Neuropathic Pain.' NEUROPHARMACOLOGY 2011, pages 626 - 632, XP028129786

## Description

### Technical field

This invention relates to biotechnology field. More specifically, this invention involves substances for treatment or relief of pain.

### Background

Pain is one sort of feeling that is different from touch, pressure, heat and cold. Terms such as sharp pain, dull pain, aching pain, stabbing pain, cutting pain or burning pain are often used by patients to describe pain. Generally, the pain induced by nerve injury and showing hyperalgesia is referred as "neuropathic" pain, and the pain caused from the stimulating nociceptor is referred as "nociceptive" pain.

Clinically, pain can be divided into two classes: acute pain and chronic pain. Acute pain is induced by the damage of skin, body structure or internal organs and/or noxious stimulation caused by diseases, or by the abnormal function of muscle or organ that does not generate actual tissue injury. Chronic pain can be defined as one kind of pain that lasts a period of time that exceeds the common course or healing time of acute diseases, or that is associated with the chronic pathological processes that cause continuous pain, or that relapses for several months or years with certain interval. If pain still exists after treatment that should cure the disease, such pain can be regarded as chronic pain. For the purpose of this invention, chronic pain can be chronic non-palliative or recurrent. The differences between the definition of acute pain and chronic pain are not only semantic differences, but also with important clinic correlation. For example, if acute pain cannot be well-controlled, it can develop to chronic pain.

Acute pain is different from chronic pain in the aspects of etiological mechanism, pathology and diagnoses and treatment. In contrast to the transiency of acute pain, chronic pain is induced by the chronic pathological processes in body structure and internal organs, or by peripheral or central nervous system or the extended and sometimes permanent dysfunction thereof. In addition, chronic pain is sometimes attributed to psychological mechanisms and/or environmental factors.

Generally, acute pain is non-neuropathic pain and includes common diseases such as arthritis pain, musculoskeletal pain, postoperative pain and fibromyalgia. The majority of such pain is thought to be caused by soft tissue and bone injury, for example arthritis pain, musculoskeletal pain and postoperative pain, and causes inflammatory reactions in normally functional nervous system, with pain just the consequence of inflammatory process.

Chronic pain includes neuropathic pain, inflammatory pain and cancer pain, which is associated with hyperalgesia and/or allodynia, wherein hyperalgesia means elevated sensitivity to typical noxious stimulation while allodynia means elevated sensitivity to typical non-noxious stimulation.

Somatogenic pain is caused by peripheral sensory nerve injury or infection, including but not limiting to pain caused by peripheral nerve injury, herpesvirus infection, diabetes, causalgia, blood vessel or plexus avulsion, neuralgia, amputation and nodular vasculitis. Neuropathic pain can also be induced by the nerve injury caused by chronic environmental poisoning, infection of human immunodeficiency virus, hypothyroidism, uremia or vitamin deficiency. Its clinical manifestation includes inflammatory pain, osteoarthritis pain, trigeminal neuralgia, cancer pain, diabetic neuropathy, restless legs syndrome, postherpetic neuralgia, causalgia, brachial vessel plexus avulsion, occipital neuralgia, gout, phantom limb, burn and other forms of neuralgia, neurological and spontaneous pain syndrome.

Neuropathic pain is generally considered as chronic pain caused by peripheral or central nervous system injury or diseases. The diseases related to neuropathic pain include long-term peripheral or central neuron sensitization, central sensitization associated with nervous system inhibitory and/or exciting function injury as well as abnormal interaction between parasympathetic and sympathetic nervous system. Many clinical symptoms are related with neuropathic pain or form the basis of neuropathic pain, including for example diabetes, postoperative pain of amputation, lower back pain, cancer, chemical injury or toxin, other serious surgeries, peripheral nerve injury caused by traumatic injury compression, nutrition deficiency, infection such as herpes zoster and HIV, etc.

The stimulation level that makes pain get attention is referred as "pain threshold". Analgesics are the medicines that increase patients' pain threshold without loss of consciousness so as to relieve pain. In the individuals suffering hyperpathia, analgesics possess the function of resisting pain sensitivity. Currently, the prescription analgesics approved by United States Food and Drug Administration (FDA) can only be divided into two classes: opioid and anti-inflammatory drugs. Anesthetics are classified into another class. Opioid analgesics take effect by mimicking endogenous opioid substances, i.e. endorphin and enkephalin generated by the body to help to relieve pain, and interacting with the opioid receptors all over the central and peripheral nervous system to block pain. Anti-inflammatory drugs include non-selective non-steroidal anti-inflammatory analgesics and specific COX-2 inhibitor, trying to lower the inflammation generated by chemical transmitters such as prostaglandin caused by body damage.

Among all opioid analgesics, morphine is still the most widely used analgesics. However, except for its therapeutic properties, it has several disadvantages, including respiratory depression, gastrointestinal movement reduction (causing constipation), nausea and vomiting. Tolerance and physical dependence also limit the clinical use of opioid compounds. Anti-inflammatory analgesics include aspirin and other salicylic acid compounds, capable of preventing the expansion of inflammatory process and temporarily relieving pain. But the effects of the drugs above on neuropathic pain are not significant. Moreover, many anti-inflammatory drugs, particularly non-steroidal anti-inflammatory analgesics, will cause gastrointestinal side effects. Such side effects include gastrointestinal ulcers and erosion. Such generally asymptomatic symptoms will become serious enough and need to hospitalization, and even lead to death. Currently, gabapentin is the first-line clinical drugs for neuropathic pain and is widely used. Gabapentin has significant side effects, including somnolence, dizziness, unsteady walking and feelings of fatigue, which often occurs at the beginning of medication. Children will sometimes be irritable, and such side effect disappears after drug withdrawal.

According to these reality, more efficient analgesics are needed, which should be with higher safety and tolerance and without addiction. Ideal analgesics are able to relieve or eliminate patients' feelings of pain and generate analgesia during the occurrence of various pains. Its effect should be satisfying no matter oral or other administration methods are adopted, producing the minimal side effect or no side effect, and without the trends of drug tolerance and dependence.

γ-aminobutyric acid (GABA) is an important inhibitory neurotransmitter in mammal central nervous system. There are two classes of GABA receptors in natural. One is GABA_{A} receptor, one member of ligand-gated ion channel superfamily, and the other is GABA_{B} receptor, one member of G protein-coupled receptor superfamily. It is found that there are several subunits in mammal GABA_{A} receptor, including α1-6, β1-4, γ1-3, δ, ε, θ and ρ1-2, among which α subunit, β subunit and γ subunit are indispensable for forming a complete and functional GABA_{A} receptor, and α subunit is crucial for the interaction between benzodiazepine and GABA_{A} receptor. The GABA_{A} receptors that can bind benzodiazepine are mainly those containing α1, α2, α3 or α5 subunit. It is reported that GABA_{A} receptor that contains α1 subunit mainly mediates tranquillization and muscle relaxation, while GABA_{A} receptors that contain α2 and α3 subunits mainly mediate anticonvulsion function. The function of GABA_{A} receptor on pain has been widely studied, especially the fact that the agonist of GABA_{A} receptor can suppress pain in central nervous system. In 2008, it was reported that the agonist of GABA_{A} receptors that contained α2 and α3 subunits significantly suppressed neuropathic pain and inflammatory pain on spinal cord level. It is reported that GABA is able to trigger an excitatory current in rat or human peripheral nervous system, and the agonist of GABA_{A} receptor Muscimol can suppress formalin-induced pain at low dose and enhance formalin-induced pain at high dose. The function of GABA_{A} receptors on pain is still unclear. The wide range of expression of GABA_{A} receptors in central nervous system limits the application of a series of ligands on pain treatment.

The percentage of GABA_{A} receptor that contains α5 subunit (α5-GABA_{A} receptor) in mammal brain GABA_{A} receptors is less than 5%. The expression level of α5-GABA_{A} receptor in cerebral cortex is very low, while the percentage of GABA_{A} receptor in hippocampus is more than 20%. There is almost no expression in other brain regions. Previous work has suggested that α5-GABA_{A} receptor may be related with cognition (Collinson N et al., Enhanced learning and memory and altered GABAergic synaptic transmission in mice lacking the alpha 5 subunit of the GABAA receptor J Neurosci. 2002 Jul. 1;22(13):5572-80).

Considering the specific distribution and functional research of α5-GABA_{A} receptor in hippocampus, a large number of pharmaceuticals companies including Merck Sharp & Dohme are working on α5-GABA_{A} receptor. Many compounds have been synthesized gradually, particularly the compounds for α5-GABA_{A} receptor, for the treatment of cognition related diseases. A5I and MRK016 are two compounds developed by Merck Sharp & Dohme to treat cognition related diseases. A5I was found to cause renal toxicity due to its low solubility in Phase II clinical trial. There exist problems on the safety of MRK016; in Phase I clinical trial, it was found that this medicine would cause side effects such as front heaviness, headache and nausea and vomiting.

Peripheral nervous system is composed of somatic nervous system and automatic nervous system. Somatic nervous system is classified into two classes: sensory nervous system (afferent nerve) and motor nervous system (efferent nerve), which are distributed all over the body. The neuron that transmits nerve impulse from peripheral to central nervous system is referred as primary sensory neuron. In sensory nervous system, only thoracic nerves walk one by one under ribs, forming intercostal nerve to control the skin and muscle on chest wall and abdominal wall. Other sensory nerves are connected by adjacent several nerves, forming nerve plexus such as cervical plexus, brachial plexus, lumbar plexus and sacral plexus. Many nerves are derived from each plexus and distributed to the skin and muscle of neck, upper chest, upper extremities, lower extremities and perineum. The primary sensory neurons of dorsal root ganglion can be divided into different functional groups based on the cell diameter, typically into small cell, medium cell and large cell. Small and medium cells are generally thought to be cells transmitting nociception. The free nerve endings of some small and medium cells are sensitive to noxious and constant stimulation, which is referred as nociceptor. It is found that α5-GABA_{A} receptor is mainly expressed in small neurons, and the expression level elevates in neurectomy model (Xiao HS et al., Identification of gene expression profile of dorsal root ganglion in the rat peripheral axotomy model of neuropathic pain." Proc Natl Acad Sci USA. 2002 Jun 11;99(12):8360-5). WO-A-2011/020044 discloses the use of substituted triazolophthalazines, which are selective partial or full inverse agonists of GABA_{A} receptors containing the α5 subunit for the treatment of conditions that are beneficially treated by such agonist, such as pain. WO-A-03/099816 discloses imidazo-pyridine derivatives which act as inverse agonists at the α2, α3 and/or α5 subunit of the GABA_{A} receptor for use in the treatment of pain or nociception. Zeilhofer et al. disclose in Ann. Rev. Pharm. Tox., 2011, 111-113, the antihyperalgesic or analgesic actions of the α5 specific GABA_{A} receptor negative allosteric modulator α5lA-II.

### Summary

The purpose of this invention is to provide substances for treatment or relief of pain. Also disclosed is a screening method for analgesics. The invention is defined by the claims. Any subject-matter falling outside the scope of the claims is provided for information purposes only. In the first aspect, the present invention provides an inverse agonist of α5-GABA_{A} receptor or its pharmaceutically acceptable salt as defined in the claims for use to prevent, improve (relieve) or treat pain.

In one preferred embodiment, the inverse agonist of α5-GABA_{A} receptor is a ligand that selectively binds to the benzodiazepine binding site of the receptor.

In another preferred embodiment, the binding capacity of the ligand of the α5-GABA_{A} receptor to α5-GABA_{A} receptor is higher than that to the GABA_{A} receptor containing α1 or α2 or α3.

In another preferred embodiment, the efficiency of inversely agonizing α5-GABA_{A} receptor by the ligand of the α5-GABA_{A} receptor is higher than that by the GABA_{A} receptor containing α1 or α2 or α3. In one aspect of the disclosure, the inverse agonist of α5-GABA_{A} receptor is a compound having the structure shown in Formula (I) and (II) or its pharmaceutically acceptable salt: 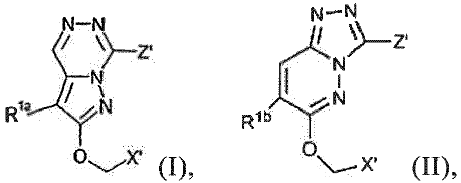
wherein R^{1a} represents halogen; or C₁₋₆ alkyl group, C₃₋₇ cycloalkyl group, C₄₋₇ cycloalkenyl group, C₆₋₈ bicycloalkyl group, C₆₋₁₀ aromatic group, C₃₋₇ heterocycloalkyl group, heteroaryl group that is defined as or contains 6 atoms, among which one, two or three atoms are nitrogen, or contains 5 atoms, among which one, two or three atoms are independently selected from oxygen, nitrogen and sulfur and the number of oxygen or nitrogen atom is no more than one, or di (C₁₋₆) alkyl amino group. Any one of these groups can be substituted with the one or more substituent groups selected from the following: halogen, R³, OR³, OC(O)R³, NR⁴R⁵, NR⁴R⁵(C₁₋₆) alkyl group, NR⁴R⁵C(O), NR⁴R⁵C(O) (C₁₋₆) alkyl group, CN, cyano (C₁₋₆) alkyl group or R⁶.
R^{1b} represents C₁₋₆ alkyl group, C₃₋₇ cycloalkyl group, C₄₋₇ cycloalkenyl group, aromatic group, C₃₋₇ heterocycloalkyl group, heterocycloaromatic group, di (C₁₋₆) alkyl amino group.
R³ represents C₂₋₆ alkyl group, C₂₋₆ alkenyl group, C₂₋₆ alkynyl group, C₃₋₆ cycloalkyl group, C₃₋₆ cycloalkyl (C₁₋₆) alkyl group, cyano (C₁₋₆) alkyl group, hydroxyl (C₁₋₆) alkyl group, and R³ can be optionally substituted with 1, 2 or 3 fluoro.
R⁴ and R⁵ independently represent hydrogen, C₁₋₆ alkyl group, C₂₋₆ alkenyl group, C₂₋₆ alkynyl group, C₃₋₆ cycloalkyl group or CF₃; alternatively, R⁴ and R⁵ and the nitrogen atom connected with both R⁴ and R⁵ form a four- to seven-membered hetero-fatty ring which contains the nitrogen atom and another atom selected from O, N and S. The ring can be substituted with one or more R³ groups.
R⁶ represents C₆₋₁₀ aromatic group, C₆₋₁₀ aromatic (C₁₋₆) alkyl group, heteroaryl group or heteroaryl (C₁₋₆) alkyl group. The heteroaryl group is defined as above; optionally, R⁶ can be substituted with one, two or three substituent groups selected from the following: halogen atom and C₁₋₄ alkyl group, C₂₋₄ alkenyl group, C₂₋₄ alkynyl group, C₁₋₄ alkoxy group, C₂₋₄ alkenoxy group and C₂₋₄ alkynoxy group, wherein each group has no substituent or can be optionally substituted with one, two or three halogen atoms.
X' represents NR⁴R⁵; alternatively, X' represents five-membered heteroaryl group that contains one, two, three or four hetero-atoms independently selected from oxygen, nitrogen and sulfur and the number of oxygen atom plus the number of nitrogen atom is no more than one, or six-membered heteroaryl group that contains one, two or three nitrogen atoms. The five-membered or six-membered heteroaryl group can be optionally condensed with benzene ring or pyridine ring, and the heteroaryl group can be optionally substituted with R^{w} and/or R^{y} and/or R^{z}. R^{w} represents halogen, R³, OR³, OC(O)R³, C(O)OR³, NR⁴R⁵, NR⁴C(O)R⁵, OH, tri (C₁₋₆ alkyl) silyl C₁₋₆ alkoxy C₁₋₄ alkyl group, CN or R⁶. R^{y} represents halogen, R³, OR³, OC(O)R³, NR⁴R⁵, NR⁴C(O) R⁵, NR⁴ R⁵(C₁₋₆) alkyl group or CN, and R^{z} represents R³, OR³ and OC(O)R³. Provided that When X' is pyridine derivative, the pyridine ring should be optionally N-oxide form. And provided that When X' is tetrazole derivative, it should be protected by C₁₋₄ alkyl group; or X' should be phenyl group that is substituted with one, two or three groups independently selected from the following groups: halogen, cyanogroup, C₁₋₆ alkyl group, C₂₋₆ alkenyl group, C₂₋₆ alkynyl group and C₃₋₆ cycloalkyl group.
Z' represents five-membered heteroaryl group that contains one, two or three hetero-atoms independently selected from oxygen, nitrogen and sulfur and the number of oxygen or nitrogen atom is no more than one. Provided that when one atom is oxygen or sulfur, there should be at least one nitrogen atom. Alternatively, Z' represents six-membered heteroaryl group that is not pyrazine and contains two or three nitrogen atoms. Any one of the heteroaryl group can be optionally substituted with one or more substituent group selected from following: halogen, R³, OR³, OC(O)R³, NR⁴R⁵, NR⁴R⁵(C₁₋₆) alkyl group, NR⁴R⁵C(O), NR4R⁵C(O)(C₁₋₆) alkyl group, CN, cyano (C₁₋₆) alkyl group or R⁶.

In another aspect of the disclosure, the inverse agonist of α-GABA_{A} receptor also includes: the isomer or precursor of the compound having the structure shown in Formula (I) and (II).

In another aspect of the disclosure, the inverse agonist of α5-GABA_{A} receptor is a compound having the structure shown in Formula (III or V) or its pharmaceutically acceptable salt: 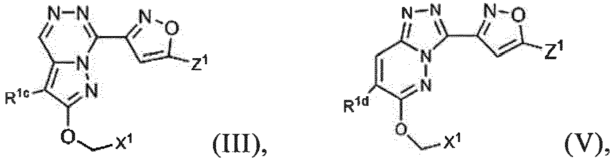 wherein R^{1c} represents bromine, thienyl group, tert-butyl group, phenyl group and furan group; R^{1d} represents aromatic group, C₁₋₆ alkyl group, C₃₋₇ cycloalkyl group; X¹ represents five-membered heteroaryl group that contains one, two or three nitrogen atoms and there is at most one oxygen or sulfur heteo-atom, or six-membered heteroaryl group that contains one, two or three nitrogen atoms. Any one of the five- or six-membered heteroaryl group can be substituted with one or more substituent groups selected from the following: C₁₋₆ alkyl group, amino group, pyridyl group, CF₃, aromatic (C₁₋₆) alkyl group, pyridyl (C₁₋₆) alkyl group, halogen, cyanogroup, cyano (C₁₋₆) alkyl group, hydroxymethyl group, hydroxyl group or the keto tautomer thereof. Z¹ represents C₁-C₄ alkyl group, C₂-C₄ alkenyl group, C₂-C₄ alkynyl group and C₁-C₄ alkoxy group substituted with hydroxyl group, halogen, hydroxyl group or amino group.

In another aspect of the disclosure, the inverse agonist of α5-GABA_{A} receptor also includes: the isomer or precursor of the compound having the structure shown in Formula (III or V).

In another aspect of the disclosure, the inverse agonist of α5-GABA_{A} receptor is a compound shown as following or its pharmaceutically acceptable salt. According to the invention, the compound is selected from the first three compounds on the first row shown below, as claimed in claim 1.

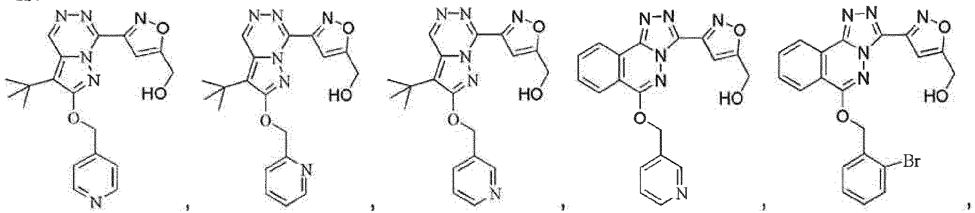

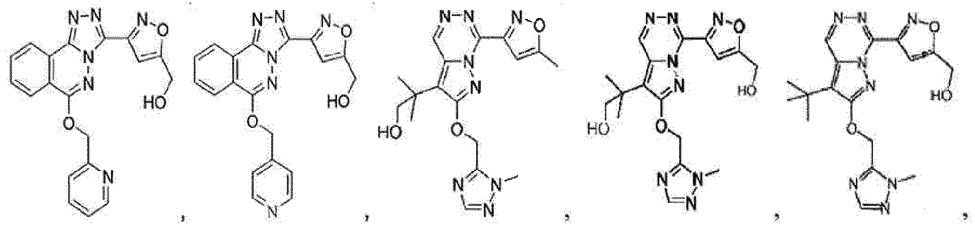

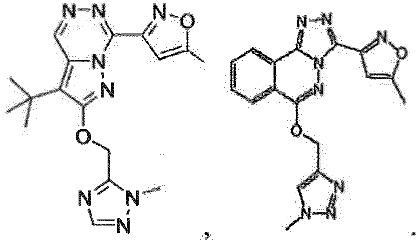

In another preferred embodiment, more preferably, the inverse agonist of α5-GABA_{A} receptor is a compound shown as following or its pharmaceutically

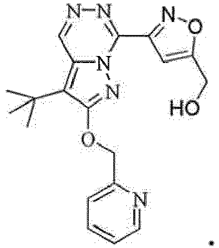

In another aspect of the disclosure, the inverse agonist of α5-GABA_{A} receptor is a compound having the structure shown in Formula (VI) or its pharmaceutically acceptable salt: 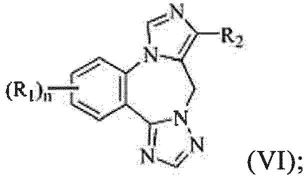 wherein R₁ represents halogen, C₁₋₇ alkyl group, C₂₋₇ alkynyl group, cycloalkyl group, C₁₋₇ alkoxy group, OCF₃, -NHR, -NHC(O)R or -NHSO2R; R represents hydrogen, C₁₋₇ alkyl group, C₁₋₇ alkyl group substituted with halogen, heteroaryl group, -(CH₂)ₙO-C₁₋₇ alkyl group or -NH-C₁₋₇ alkyl group; R₂ represents halogen, hydrogen, -C(O)O(C₁₋₄) alkyl group, C(O)NHCH₂CCH, C(O)NHCH₂CH₂, SO₂CH₂CH₃, C₁₋₇ alkyl group, C₁₋₇ alkyl group substituted with halogen, cyanogroup, C₃₋₆ cycloalkyl group; n is 0, 1, 2 or 3.

In another aspect of the disclosure, the inverse agonist of α5-GABA_{A} receptor is a compound shown as following or its pharmaceutically acceptable salt. The compound is:

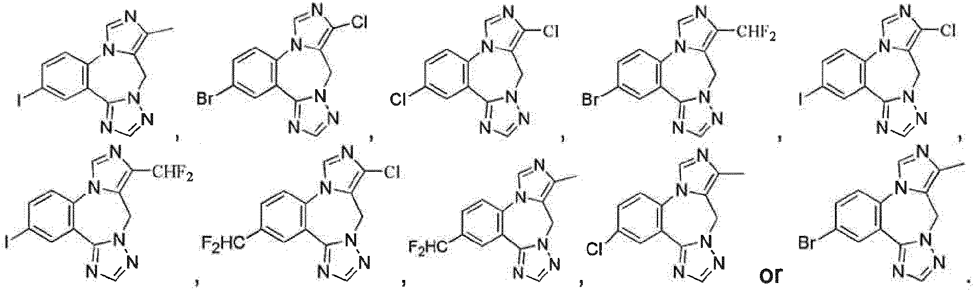

In another aspect of the disclosure, the inverse agonist of α5-GABA_{A} receptor is a compound shown as following or its pharmaceutically acceptable salt. The compound is:

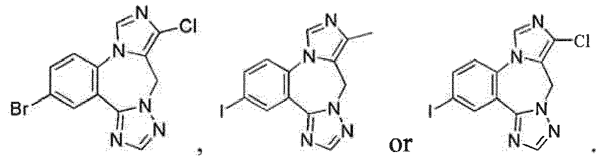

In another preferred embodiment, the inverse agonist of α5-GABA_{A} receptor can bind the α5-GABA_{A} receptor expressed in peripheral nervous system but cannot effectively bind the α5-GABA_{A} receptor expressed in central nervous system.

In another preferred embodiment, the pain is peripheral nerve associated chronic pain.

In another preferred embodiment, the chronic pain is neuropathic pain, inflammatory pain and cancer pain.

In another preferred embodiment, the pain is as defined in claim 5. Pain or diseases associated with pain include: headache, facial pain, neck pain, shoulder pain, back pain, thoracic pain, abdominal pain, dorsopathy, waist pain, lower limb pain, muscle and bone pain, body pain, vascular pain, gout, arthritis pain, somatoform disorder associated pain, visceral pain, the pain caused by infectious diseases such as AIDS and postherpetic neuralgia, pain associated with multiple bone pain, sickle cell anemia, autoimmune disease, multiple sclerosis or inflammation acute or chronic inflammatory pain, cancer pain, neuropathic pain, injury or surgery caused pain, cancer pain, nociceptive pain, diabetes, peripheral neuropathies, postherpetic neuralgia, trigeminal neuralgia, waist or cervix radiculopathy, glossopharyngeal neuralgia, autonomic nerve reflex pain, reflex sympathetic dystrophy, nerve root avulsion, cancer, chemical injury, toxin, nutrition deficiency, virus or bacteria infection, degenerative osteoarthropathy or the combination thereof.

In another aspect, the present disclosure provides a method for screening drugs used to prevent, improve or treat pain. The method includes:
(1) providing a system containing α5-GABA_{A} receptor, wherein the α5-GABA_{A} receptor possesses complete GABA_{A} receptor function.
(2) administrating candidate substances to the system of step (1), observing the binding between candidate substance and α5-GABA_{A} receptor; if one candidate substance is capable of binding α5-GABA_{A} receptor, then the candidate substance is a potential drug for preventing, relieving or treating pain.

In an aspect of the disclosure, said binding with α5-GABA_{A} receptor is: selective binding with benzodiazepine binding site.

In another aspect of the disclosure, the candidate substance cannot efficiently bind to the α5-GABA_{A} receptor expressed in central nervous system.

In another aspect of the disclosure, the candidate substance is a substance that pass in small amounts or does not pass the blood brain barrier of animals. For the tissue distribution in animal body, the distribution of candidate compound in brain is less than 50% of that in plasma.

In another aspect of the disclosure, the α5-GABA_{A} is composed of α5 subunit, β subunit such as β2 and β3 and γ subunit such as γ1 and γ3.

In another aspect of the disclosure, the step (2) includes observing the status of candidate substance inhibiting the current induced by GABA through GABA_{A} receptor (e.g. inhibiting for more than 20%, preferably inhibiting for more than 40%, and more preferably inhibiting for more than 50%). If the candidate substance can inhibit the current induced by GABA through GABA_{A} receptor, then the candidate substance is a potential drug for preventing, relieving or treating pain.

In another aspect of the disclosure, the step (1) also includes a step of setting up a control group. The control group is selected from: the system containing (e.g. expressing) the GABA_{A} receptor that contains α1 subunit, the system containing (e.g. expressing) the GABA_{A} receptor that contains α2 subunit and/or the system containing (e.g. expressing) the GABA_{A} receptor that contains α3 subunit. The GABA_{A} receptor in the control group possesses complete GABA_{A} receptor function. And in step (2), the method includes detecting the binding between candidate substance and α5-GABA_{A} receptor in the system containing (e.g. expressing) α5-GABA_{A} receptor, and comparing it with the control group. If the binding inhibition constant Ki (nM) (a physical constant representing the binding capacity between antagonist and receptor; the smaller the number is, the stronger the binding capacity is) between candidate substance and α5-GABA_{A} receptor is statistically less than (e.g. less than 20%, preferably less than 40%, more preferably less than 60%, more preferably less than 80%) the binding inhibition constant Ki between candidate substance and the GABA_{A} receptor that contains α1, α2 or α3 subunit, then the candidate substance is a potential drug for preventing, relieving or treating pain.

In another aspect of the disclosure, the candidate substance cannot efficiently inhibit the binding between the ligand of α5-GABA_{A} receptor and α5-GABA_{A} receptor in central nervous system (such binding can be identified by for example isotope labeling method). After administrating the experimental animals with a dose of candidate substance and known specific ligand of α5-GABA_{A} receptor, the specific ligand can be labeled (for example by isotope). Then whether the candidate substance can inhibit the distribution of known specific ligand of α5-GABA_{A} receptor in central nervous system is detected. If the candidate substance is capable of inhibiting the distribution of known specific ligand of α5-GABA_{A} receptor in central nervous system to less than 60%, then the candidate substance is a potential drug for preventing, relieving or treating pain.

In another aspect of the disclosure, the candidate substance cannot efficiently pass the blood brain barrier. If the distribution proportion of the candidate substance in brain and plasma is less than 50%, then the candidate substance is a potential drug for preventing, relieving or treating pain.

In another aspect of the disclosure, the step (1) also includes a step of setting up a control group. The control group is selected from: the system containing (e.g. expressing) the GABA_{A} receptor that contains α1 subunit, the system containing (e.g. expressing) the GABA_{A} receptor that contains α2 subunit and/or the system containing (e.g. expressing) the GABA_{A} receptor that contains α3 subunit. The GABA_{A} receptor in the control group possesses complete GABA_{A} receptor function. And in step (2), the method includes: using electrophysiology methods to detect the inhibition of candidate substance on the current induced by GABA through GABA_{A} receptor. If the candidate substance can significantly inhibit (e.g. more than 20%, preferably more than 40%, more preferably more than 50%) the α5-GABA_{A} receptor mediated current, and meanwhile cannot significantly inhibit the current induced by the GABA_{A} receptor that contains α1, α2 or α3 subunit, then the candidate substance is a potential drug for preventing, relieving or treating pain.

In another aspect of the disclosure, the candidate substance includes (but not limits to): small molecular compound such as the inverse agonist of γ-aminobutyric acid A, polypeptide and ligand.

In another aspect of the disclosure, the system is selected from: cellular system (or cell culture system) such as dorsal root ganglion cell, subcellular system, solution system, tissue system, organ system and animal system. In one aspect of the disclosure, the method also includes conducting further cellular and/or animal experiments on obtained potential drugs to further select and determine the really useful substances for preventing, relieving or treating pain.

In another aspect, the present disclosure provides a method for preventing, relieving or treating pain, including the administration of safe and effective amount of the inverse agonist of α5-GABA_{A} receptor or its pharmaceutically acceptable salt to the required patients.

In a preferred embodiment, the inverse agonist of α5-GABA_{A} receptor or its pharmaceutically acceptable salt can be administrated orally, by subcutaneous injection, by transdermal patch, intranasally or by automated injection device.

### Brief description of the figures

Figure 1 . Under the condition of spared nerve injury (SNI), the expression of the α5 subunit protein of GABA_{A} receptor was elevated in dorsal root ganglion.
   A. The expression of the α5 subunit protein of GABA_{A} receptor measured by immunohistochemistry experiments.
   B. The protein level of the α5 subunit of GABA_{A} receptor measured by western blot.
Figure 2 . The expression of the α5 subunit protein of GABA_{A} receptor was elevated in dorsal root ganglion in the inflammatory pain model induced by Complete Freund's adjuvant (CFA).
   A. The protein expression of the α5 subunit of GABA_{A} receptor measured by immunohistochemistry experiments.
   B. The protein level of the α5 subunit of GABA_{A} receptor measured by western blot.
Figure 3 . The α5 subunit protein of GABA_{A} receptor in dorsal root ganglion could be transported to sciatic nerve.
   A. The expression of the α5 subunit protein of GABA_{A} receptor measured by immunohistochemistry experiments.
   B. The protein level of the α5 subunit of GABA_{A} receptor in sciatic nerve, dorsal root ganglion cell body, dorsal root nerve and spinal dorsal horn measured by western blot.
Figure 4. (reference) In SNI model, the neuropathic pain could be efficiently suppressed by the administration of MRK016 or MRK016-M3.
   A. At 7 days after the establishment of SNI model, the mechanical pain threshold of different time after subcutaneous injection of MRK016.
   B. The suppression effect of MRK016-M3 on neuropathic pain measured by dose-dependent assays.
   C. The analysis of the effectiveness and the effect dose of Gabapentin on treating neuropathic pain.
   D. The rats that were at 10 days after the establishment of SNI model were used as experimental animals and MRK016-M3 was injected to the feet of rats to conduct the effectiveness analysis of MRK016-M3 on treating neuropathic pain.
   E. The siRNA specific to the mRNA of GABA_{A} α5 subunit was intrathecally injected, the expression of α5-GABA_{A} receptor in dorsal root ganglion was examined by western blotting.
   F. Intrathecally injection of the siRNA specific to the mRNA of GABA_{A} α5 subunit significantly increased the pain threshold, and control siRNA did not have obvious analgesic effect.
   G. Under the condition of intrathecally injecting the siRNA specific to the mRNA of GABA_{A} α5 subunit, MRK016-M3 did not significantly increase the nociceptive threshold; and under the condition of injecting control siRNA, MRK016-M3 significantly increased the pain threshold.

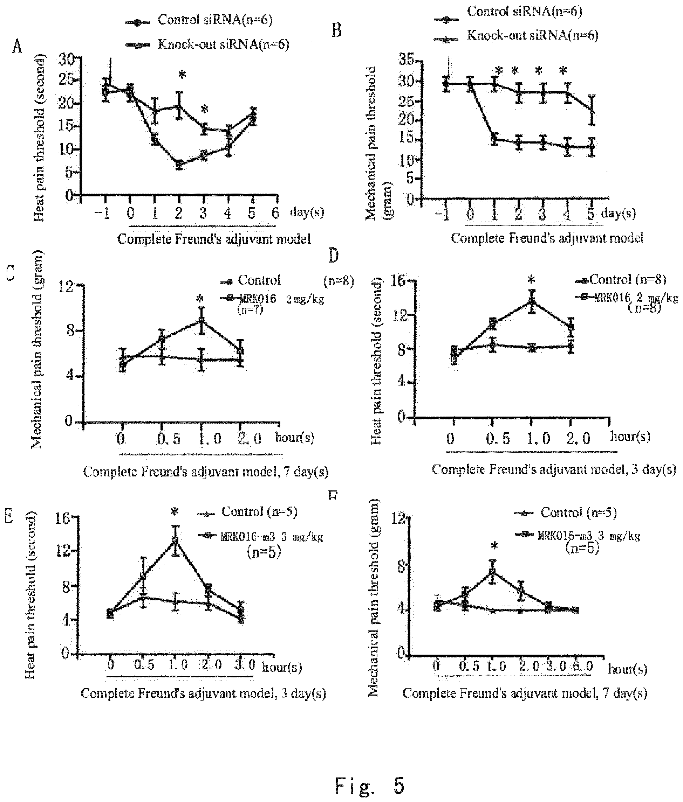

Figure 5. (reference) In CFA model, administration of MRK016 or MRK016-M3 suppressed the nociceptive response of rats.
   A. Firstly the heat pain threshold and mechanical pain threshold of rats was measured; then the siRNA specific to the mRNA of GABA_{A} α5 subunit or control siRNA was injected on the same day; 100 microliter CFA to the right feet of rats was injected on the second day; then the heat pain threshold of rats was measured everyday.
   B. Firstly the heat pain threshold and mechanical pain threshold of rats was measured; then the siRNA specific to the mRNA of GABA_{A} α5 subunit was injected on the same day; 100 microliter CFA to the right feet of rats was injected on the second day, then the mechanical pain threshold of rats was measured everyday.
   C. At 7 days after the establishment of inflammatory pain model, MRK016 (2 mg/kg body weight) was intraperitoneally injected, and the compound was found to be capable of efficiently suppress mechanical pain.
   D. At 3 days after the establishment of inflammatory pain model, MRK016 (2 mg/kg body weight) was intraperitoneally injected, and the compound was found to be capable of efficiently suppress heat pain.
   E. Heat pain was measured at 3 days after the establishment of inflammatory pain model, and the results indicated that MRK016-M3 significantly suppressed pain 1 hour after administration.
   F. Mechanical pain was measured at 7 days after the establishment of inflammatory pain model, and the results indicated that MRK016-M3 significantly suppressed pain 1 hour after administration.

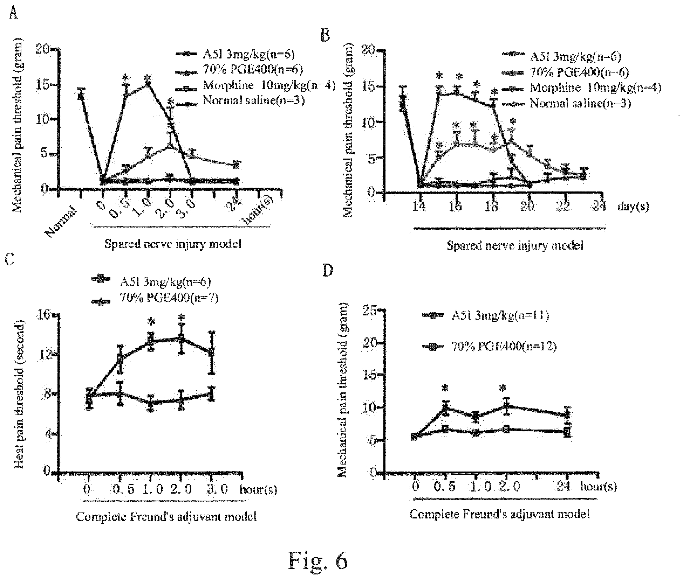

Figure 6. (reference) The treatment of the ligand of α5-GABA_{A} receptor A5I on neuropathic pain and inflammatory pain.
   A. The analgesic effect of α5IA in SNI model was measured. α5IA (3 mg/kg body weight) significantly suppressed neuropathic pain. Morphine (10 mg/kg body weight) was used as positive control.
   B. Continuous injection of α5IA did not generate tolerance response. At 14 days after the establishment of SNI model, continuous injection was conducted for 6 days, and nociceptive response was measured 1-3 hours after administration, respectively.
   C. Intraperitoneal injection of A5I (3 mg/kg body weight) also could efficiently treat heat pain 3 days after the establishment of inflammatory pain model.
   D. Intraperitoneal injection of A5I (3 mg/kg body weight) also could efficiently treat mechanical pain 7 days after the establishment of inflammatory pain model.

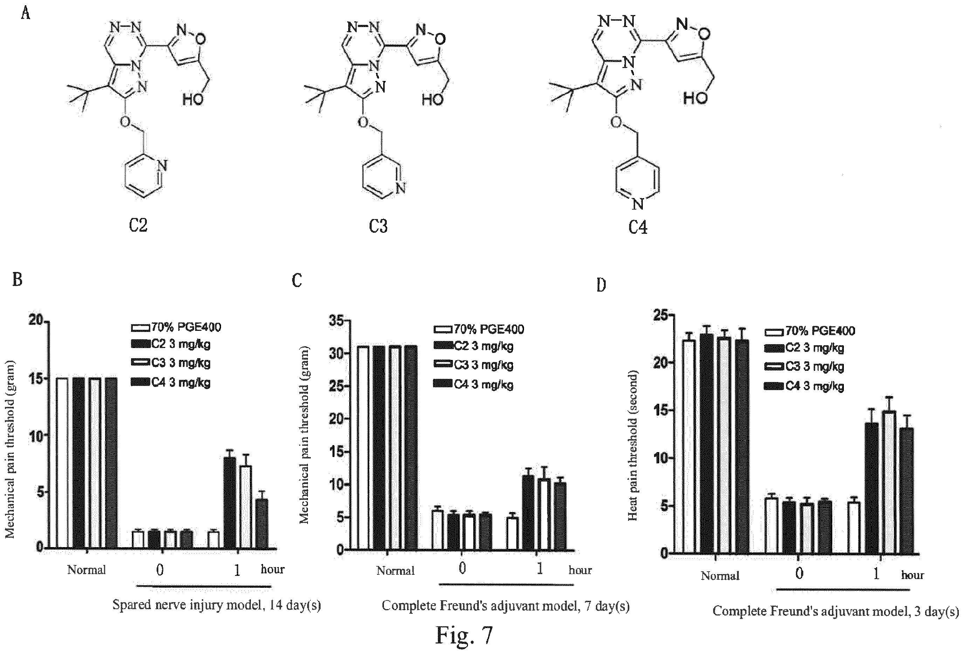

Figure 7. (according to the invention) The treatment of different ligands (C2, C3 and C4) of α5-GABA_{A} receptor on neuropathic pain and inflammatory pain.
   A. The chemical structural formula of different ligands (C2, C3 and C4).
   B. At 14 days after SNI model, C2, C3 or C4 was subcutaneously injected respectively and the analgesic effect of this series of drugs was measured respectively 1 hour later; the result showed that all of the drugs in this series could efficiently treat pain.
   C. At 7 days after the establishment of inflammatory pain model, C2, C3 or C4 was subcutaneously injected respectively and the analgesic effect of this series of drugs on mechanical pain was measured respectively 1 hour later; the result showed that all of the drugs in this series could efficiently treat pain.
   D. At 3 days after CFA model, C2, C3 or C4 was subcutaneously injected respectively and the analgesic effect of this series of drugs on heat pain was measured respectively 1 hour later; the result showed that all of the drugs in this series could efficiently treat chronic pain.

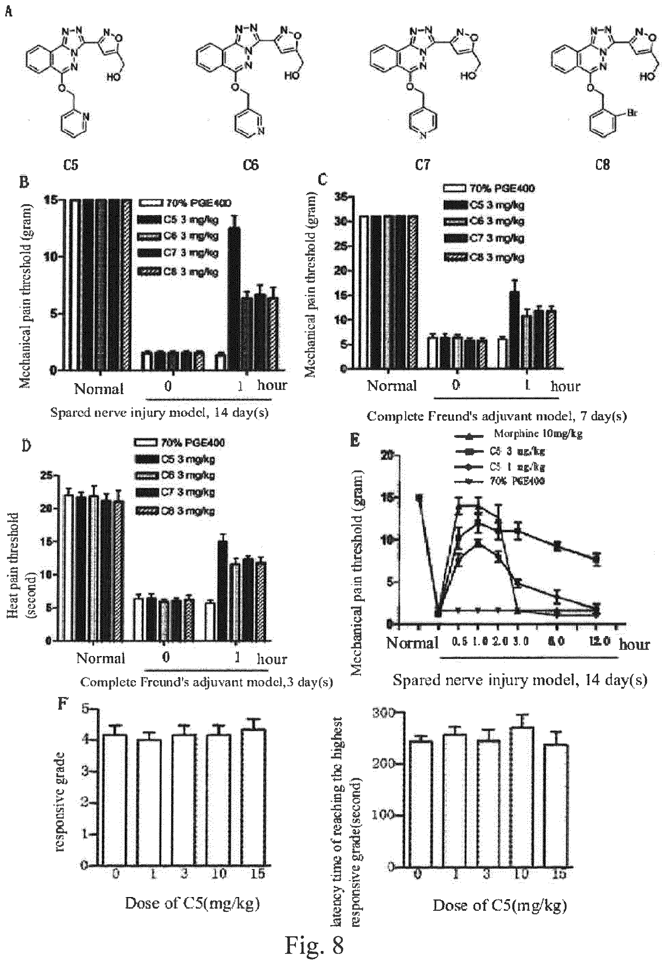

Figure 8. (reference) The treatment of different ligands (C5, C6, C7 and C8) of α5-GABA_{A} receptor on neuropathic and inflammatory pain.
   A. The chemical structural formula of different ligands (C5, C6, C7 and C8).
   B. At 14 days after SNI model, C5, C6, C7 or C8 was subcutaneously injected respectively and the analgesic effect of this series of drugs was measured respectively 1 hour later; the result showed that all of the drugs in this series could efficiently inhibit nociceptive response.
   C. At 7 days after CFA model, C5, C6, C7 or C8 was subcutaneously injected respectively and the analgesic effect of this series of drugs on mechanical pain was measured respectively 1 hour later; the result showed that all of the drugs in this series could efficiently inhibit nociceptive response.
   D. At 3 days after CFA model, C5, C6, C7 or C8 was subcutaneously injected respectively and the analgesic effect of this series of drugs on heat pain was measured respectively 1 hour later; the result showed that all of the drugs in this series could efficiently inhibit nociceptive response.
   E. At 14 days after SNI model, after subcutaneous injection C5 at the dose of 1 mg/kg or 3 mg/kg, the analgesic effect of C5 compound was detected at different time points and the result showed that C5 efficiently could inhibit nociceptive response at indicated time points. Morphine was used as positive control, subcutaneously injected at the dose of 10 mg/kg.
   F. There is no effect of compound C5 on pentetrazol-induced epilepsy. In this assay, different doses of compound C5 were subcutaneously injected at first. The doses were 1 mg/kg, 3 mg/kg, 10 mg/kg and 15 mg/kg, respectively. After 0.5 hour, 100 mg/kg pentetrazol was injected respectively. The results indicated that there was no effect of different doses of compound C5 on the responsive grade and latency time of the pentetrazol induced epilepsy.

### Detailed description

After indepth study, the present inventors reveal a new method for treating pain. The peripheral nerve specific inverse agonist of α5-GABA_{A} receptor can be used to treat pain.

The α5 subunit protein of GABA_{A} receptor disclosed herein is mainly transported to peripheral plexus or sciatic nerve, and the expression of this gene increased in SNI model and CFA induced inflammatory pain model. The inventors hypothesize that those inverse agonist of GABA_{A} receptor that pass in small amounts or does not pass blood brain barrier is capable of binding to α5-GABA_{A} receptor in peripheral nerve to suppress neuropathic pain and inflammatory pain. Considering that the α5 subunit of GABA_{A} receptor is mainly expressed in nervous system, such compound is the analgesics with theoretically no or less side effect.

### Terms

As used herein, "γ-aminobutyric acid A receptor that contains α5 subunit", "α5-GABA_{A} receptor" and "GABA_{A} receptor that contains α5 subunit" can be used interchangeably.

As used herein, "γ-aminobutyric acid A receptor" and "GABA_{A} receptor" can be used interchangeably.

As used herein, "ligand of (specific to) the GABA_{A} receptor that contains α5 subunit" is defined as a compound that binds α5-GABA_{A} receptor. The compound can adjust pain reaction.

As used herein, "inverse agonist of (specific to) the GABA_{A} receptor that contains α5 subunit" represents a compound that binds α5-GABA_{A} receptor. The compound can inhibit the function of GABA on α5-GABA_{A} receptor.

As used herein, "treatment" also includes preventive administration, preventing or eliminating the diseases after the establishment of the diseases.

As used herein, "patient" is defined as any warm-blooded animal, including but not limiting to mouse, cavy, dog, horse or human. Preferably, the patient is human.

As used herein, "acute pain" is defined as the pain caused by the injury of skin, body structure or internal organs and/or noxious stimulation of the diseases, or the pain caused by the abnormal function of muscle or internal organs that does not produce real tissue injury.

As used herein, "chronic pain" is defined as the pain that lasts a period of time that exceeds the common course or healing time of acute diseases, or that is associated with the chronic pathological processes that cause continuous pain, or that relapses for several months or years with certain interval. If pain still exists after treatment that should cure the disease, such pain can be regarded as chronic pain. The time duration that the pain lasts depends on the nature of pain and the treatment process associated with pain. If the pain exceeds common treatment process, then this pain is chronic. Chronic pain includes but not limits to headache, facial pain, neck pain, shoulder pain, thoracic pain, abdominal pain, back pain, waist pain, lower limb pain, muscle and bone pain, somatoform disorder associated pain, visceral pain, painful diabetic neuropathy, vascular pain, gout, arthritis pain, cancer pain, autonomic nerve reflex pain, the pain caused by infectious diseases such as AIDS and herpes zoster, the pain caused by autoimmune disease such as rheumatism, the pain caused by acute or chronic inflammation, postoperative pain and post-burning pain.

The drugs disclosed by this invention can efficiently treat the chronic pain defined as above, and the drugs disclosed by this invention can be used to treat hyperalgia accompanied with other diseases, including hyperalgesia, allodynia, algesia enhancement and pain memory enhancement. This invention will improve the treatment of pain.

As used herein, "headache" can be divided into primary headache and secondary headache. Primary headache includes tension headache, migraine headache and cluster headache, and secondary headache is caused by other diseases. Headache is caused when pain sensitive tissue on head and face undergo lesion or get stimulated. These pain sensitive tissues are distributed on scalp, face, oral cavity and throat. They are mainly muscles and vessels in head with abundant nerve fibers and sensitive to pain, therefore headache is caused when these tissues are injured.

As used herein, "facial pain" includes but not limits to trigeminal neuralgia, atypical facial pain, facial palsy and facial spasm.

As used herein, "trigeminal neuralgia" is a unique chronic painful disease, also referred as tic douloureux, representing transient, paroxysmal and repeated electric shock-like severe pain in trigeminal nerve area, or accompanied with ipsilateral facial spasm. Trigeminal neuralgia can be divided into two classes: primary and secondary. Primary trigeminal neuralgia means no neurological sign is found clinically and no organic disease is detected. Secondary trigeminal neuralgia means neurological signs are found clinically and organic diseases such as tumor and inflammation are detected.

As used herein, "atypical facial pain" represents pain caused by various diseases, appearing as burning pain, non-intermittent and independent of particular action or stimulation. The pain is often bilateral and exceeds the area of trigeminal nerve to even cervical skin. The etiology can be the stimulation of nasosinusitis, malignant tumor, jaw and skull base infection or injured trigeminal nerve.

As used herein, "neck pain, back pain, shoulder pain" represent the pain caused by acute or chronic muscle strain and bone joint degeneration and injury. The common diseases that cause neck, shoulder and upper limb pain include cervicoshoulder myofascitis, neck desmitis, cervical spondylopathy, scapulohumeral periarthritis, thoracic outlet syndrome, external humeral epicondylitis, etc. Alternatively, these terms represent the pain cause by autoimmune diseases rheumatoid arthritis, ankylosing spondylitis and rheumatic arthritis. Other diseases that can cause neck pain, back pain and shoulder pain are tumors on neck and shoulder, neuritis, arteriovenous disease and various infections as well as referred pain induced by lesions of thoracic and abdominal organs.

As used herein, "thoracic, abdominal, and back pain" represent the pain caused by diseases in thoracic and abdominal organs and thoracic and abdominal wall tissues, including but not limiting to intercostal neuralgia, intercostal chondritis, angina pectoris, abdominal pain (acute abdominal organ pain) and waist and back myofascial pain syndrome.

As used herein, "waist pain, lower limb pain" represent low back, lumbosacral, sacroiliac, hip, buttocks and lower limb pain. Generally, waist and lower limb pain is not independent disease, but the common feature of various diseases, with diverse clinical manifestation and complex etiology. Such pain is mainly induced by degeneration and injury, including but not limiting to the pain involving lumbar disc herniation, acute lumbar sprain, ischialgia, osteoporosis, third lumbar trans-verse process syndrome, piriformis syndrome, knee osteoarthritis, coccygodynia and calcanodynia.

As used herein, "muscle and bone pain" includes but not limits to myofascial pain, trauma-caused pain and chronic regional pain syndrome.

As used herein, "painful diabetes" represents the pain caused by nerve injury concurrent with diabetes. The nerve injury in diabetes is caused at least partly by blood flow reduction and hyperglycemia. Some diabetes patients do not suffer neuropathy, while others suffer this disease at early stage. Diabetic neuropathy can be divided into mononeuropathy that involves one or several lesion sites and systemic polyneuropathy. The polyneuropathy can be dispersive and symmetrical, generally and mainly involving mode of sensation (Merrit's Textbook of Neurology, the 9^{th} version). The manifestation of diabetic neuropathy includes plant nerve dysfunction, and cause dysregulation involving heart, smooth muscle and gland, resulting in hypotension, diarrhea, constipation and impotence. Diabetic neuropathy often develops in stages. The early stage takes place in nerve ending area. Plant neuropathy or sensory neuropathy occurs in feet and brain neuropathy occurs in face and periocular area with intermittent pain and the sense of tingling. In the following stages, the pain become more severe and occurs more frequently. Finally, when analgesia happens in one area, the disease develops into painless neuropathy. Due to lack of pain as the sign of injury, the risk of severe tissue damage is greatly increased.

As used herein, "visceral pain" includes but not limits to the pain of inflammatory bowel syndrome (IBS), with or without chronic fatigue syndrome (CFS), inflammatory bowel disease (IBD) and interstitial cystitis.

As used herein, "vascular pain" represents the pain generated by the following one or more factors. Firstly, improper perfusion of tissue, resulting in temporary or continuous ischemia, e.g the ischemia in limb muscles during physical exercise. Secondly, delayed change, e.g. ulcer or gangrene in skin or abdominal organs. Thirdly, the sudden and accelerated change of diameter of great vessels, e.g. the change of arterial aneurysm. Fourthly, aortic rupture, resulting in blood spillover and the stimulation of nociceptive fibers in peritoneum or pleura parietal layers. Fifthly, strong cramp caused by the severe stimulation of artery endothelium by intra-arterial injection. Sixthly, the damage of venous return, leading to a large number of edema of rapidly expanded fascia compartment (Bonica's Management of Pain, Volume 1 (the 2nd version)). The examples include but not limit to arteriosclerosis obliterans, thromboangiitis angiitis, acute arterial closure, embolism, congenital arteriovenous aneurysm, vasospasm diseases, Rayaud's disease, acrocyanosis, acute venous closure, thrombophlebitis, varicosity and lymphedema.

As used herein, "cancer pain" represents the pain occurs during the development process of malignant tumor. Currently, it is thought that there are three mechanisms of cancer pain, i.e. the pain caused directly by cancer development, the pain caused after cancer treatment and the concurrent painful diseases of cancer patients.

As used herein, "autonomic nerve reflex pain" represents the pain caused by "reflex sympathetic dystrophy". For reflex sympathetic dystrophy, after the body suffers acute or chronic injury, severe ambulatory pain occurs and the body is sensitive to the sense of touch and pain, probably accompanied with edema and blood disorder, following symptoms like skin and musculoskeletal nutrition dystrophia and atrophy.

As used herein, "postoperative pain" represents a complex physiological response of body to the disease itself and the tissue injury caused by operation, showing an unpleasant psychological and behavior experience.

As used herein, "arthritis pain" includes but not limits to the pain caused by osteoarthritis, rheumatoid arthritis, joint ankylosing spondylitis, psoriatic arthropathy, gout, pseudo gout, infectious arthritis, tendinitis, bursitis, bone damage and joint soft tissue inflammation.

As used herein, "postherpetic neuralgia" represents the subcutaneously long-standing severe pain in rash site after the healing of the rash of herpes zoster.

As used herein, "nociceptive pain" represents the pain caused by the tissue injury delivered by nociceptors, or the pain caused by the extended excitement of nociceptors. The pain caused by the extended excitement of nociceptors can be induced by both the persisting noxious stimulation of nociceptors and the sensitization thereof, or they can be induced by these factors and extended by their persistence, various reflex mechanisms and other factors.

As used herein, "algesia" represents the neuromechanism for detecting noxious stimulation. Algesia involves two steps: the transduction of noxious stimulation by peripheral nerve ending and delivering these signals to central nervous system.

As used herein, the term "alkyl group" represents saturated and straight chain or branched chain aliphatic hydrocarbon group. For example, alkyl group includes but not limits to methyl group, ethyl group, propyl group, isopropyl group, n-butyl group, isobutyl group, tert-butyl group. The "alkyl group" is preferably C₁₋₈ alkyl group, more preferably C₁₋₆ alkyl group, more preferably C₁₋₄ alkyl group.

As used herein, the term "alkenyl group" represents the straight chain or branched chain hydrocarbon group that contains at least one carbon-carbon double bond and at least 2 carbon atoms (preferably 2-6 carbon atoms). The "alkenyl group" is preferably C₂₋₈ alkenyl group, more preferably C₂₋₆ alkenyl group, more preferably C₂₋₄ alkenyl group.

As used herein, the term "alkynyl group" represents the straight chain or branched chain hydrocarbon group that contains at least one carbon-carbon triple bond and at least 2 carbon atoms (preferably 2-8 carbon atoms, more preferably 2-6 carbon atoms, more preferably 2-4 carbon atoms).

As used herein, the term "halogen" represents F, Cl, Br or I, especially F, Cl or Br.

As used herein, the term "alkoxy group" represents the alkyl group that contains oxygen, e.g. methoxy group, ethoxy group, propoxy group and isopropoxy group. It contains preferably 2-8 carbon atoms, more preferably 2-6 carbon atoms, more preferably 2-4 carbon atoms.

As used herein, the term "cycloalkyl group" represents the cyclic alkyl group that contains 3 to 7 carbon atoms, e.g. cyclopropyl group, cyclopentyl group, cyclohexyl group and cycloheptyl group.

As used herein, the term "cycloalkenyl group" is defined as "cycloalkyl group", and contains at least one unsaturated carbon-carbon double bond.

As used herein, the term "aromatic group" represents aromatic system. It can be monocyclic aromatic group or polycyclic aromatic group that is originally condensed and connected, an aromatic system that makes at least part of condensed and connected rings conjugated. Aromatic group includes (but not limits to) phenyl group and naphthyl group.

As used herein, the term "heterocyclic ring" represents stable C₄₋₇ monocyclic group or stable polycyclic group, which can be saturated, partly unsaturated or unsaturated and is composed of carbon atoms and 1-4 hetero atoms selected from the following: N, O and S atom. N and S atom can be oxidized. Heterocyclic ring also includes any polycyclic ring. Any of above heterocyclic rings can be condensed to aromatic rings.

As used herein, the term "hetero aromatic group" represents the aromatic group of heterocyclic ring.

As used herein, the term "isomer" includes conformational isomer, optical isomer (e.g. enantiomer and diastereoisomer) and geometric isomer (e.g. cis-trans isomer).

### The inverse agonist of the γ-aminobutyric acid A receptor that contains α5 subunit and its application

The inverse agonist of α5-GABA_{A} receptor has been studied for decades, with new compounds gradually synthetized. Previous studies thought that it was capable of treating learning memory (cognition) associated diseases.

After studying in depth, the inventors have found α5-GABA_{A} receptor can efficiently suppressing pain. The inverse agonist of α5-GABA_{A} receptor is the compound that can bind to α5-GABA_{A} receptor. The compound can bind to the benzodiazepine binding sites of α5-GABA_{A} receptor. The compound can be the inverse agonist of α5-GABA_{A} receptor. Preferably, the compound cannot effectively pass blood brain barrier and bind the α5-GABA_{A} receptor in central nervous system.

The α5 subunit protein of GABA_{A} receptor is expressed in dorsal root ganglion, and mainly transported to periphery. The expression of the α5 subunit protein of GABA_{A} receptor is elevated in CFA induced inflammatory pain model and SNI model. Through knocking down the expression of the α5 subunit protein of GABA_{A} receptor in dorsal root ganglion, inflammatory pain and neuropathic pain can be obviously suppressed. Moreover, administration of the inverse agonist of α5-GABA_{A} receptor can significantly suppress inflammatory pain and neuropathic pain.

The binding capacity of the inverse agonist of α5-GABA_{A} receptor to α5-GABA_{A} receptor is higher than that to the GABA_{A} receptor that contains α1 or α2 or α3 subunit.

In one aspect, the disclosure provides an inverse agonist of α5-GABA_{A} receptor, as shown in Formula (I and II): 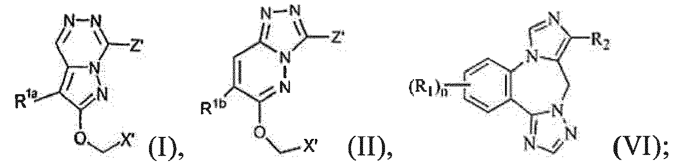 wherein the definition of each group is shown as above.

In another aspect, this disclosure provides an inverse agonist of α5-GABA_{A} receptor as shown in Formula (II), preferably shown in Formula (III) or Formula (IV): 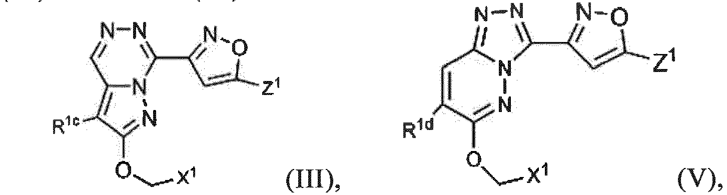 wherein the definition of each group is shown as above.

The agonist of α5-GABA_{A} receptor according to the disclosure also includes those mentioned in the following patents: WO 2011/107812 A2, WO 2010/112475 A1, WO 2010/104843 A2, WO 2010/002451 A1, WO 2010/127978 A1 , WO 2010/127968 A1, WO 2010/097368 A1, WO 2010/125042 A1, WO 2010/028769 A1, WO 2010/094669 A1, WO 2010/127976 A1, WO 2010/127975 A1, WO 2009/000662 A1, WO 2009/071464 A1, WO 2009/071476 A1, WO 2009/071477 A1, WO 2008/154438 A1, WO 2008/154442 A1, WO 2008/154447 A1, WO 2007074089 A1, WO 2007/071598 A1, WO 2007/074078 A2, WO 2010/127974 A1, WO 2007/137954 A1, WO 2007/042420 A1, WO 2007/054444 A2, WO 2007/042421 A1, WO 2007/039389 A1, WO 2007/082806 A1, WO 2006/078891 A2, WO 2006/045430 A1, WO 2006/045429 A1, WO 2006/040038-A1, WO 2006/063708 A1, WO 2005/080355 A1, WO 2005/084439 A1, WO 2005/108401 A1, WO 2004/041808 A1, WO 2004/014865 A1, WO 2004/014891 A1, WO 2004/074259 A1, WO 2004/039802, WO 2004/014865 A1, WO 2004/043930 A1, WO 2004/087137 A1, WO2004076452-A1, WO 2004/031174 A1, WO 2004031174 A1, WO 2004/107863 A1, WO2004065388-A1, WO 2003/093263 A1, WO 2003/086406 A1, WO 2003/093273 A1, WO 2003/087099 A1, WO 2003/048132 A1, WO 2003/066634 A1, WO 2003/008418 A1, WO 2003/097643 A1, WO 2003/044018 A1, WO 2003/006471, WO 2003/004018 A1, WO 2003/093272 A1, WO 2003/099816 A1, WO 2003/099817 A1, WO 2002/081474 A1, WO 2002/020480 A1, WO 2002/076987 A1, WO 2002/076983 A1, WO 2002/000623 A2, WO 2002/006285 A1, WO 2002/016363 A1, WO 2002/020492 A1, WO 2002/094834 A1, WO 2002/042305 A1, WO 2002/074773 A1, WO 2002/094834 A1, WO 2002/002557 A2, WO 2001/038331 A2, WO 2001/092257 A1, WO 2001/018001 A1, WO 2001/016103 A1, WO 2001/044250 A1, WO 2001/092258 A1, WO 2001/090108 A1, WO 2001/051492 A1, WO 2000/027849 A2, WO 2000/029412 A1, WO 2000/012505 A2, WO 2000/077008 A2, WO 2000/059905 A1, WO 1999/006400 A1, WO 1999/006399 A1, WO 1999/006401 A1, WO 1998/004560 A1, WO 1998/024435 A1, WO 1998/018792 A1, WO 1996/025948 A1, WO 1996/008494 A1, US 6511987, US 6297256, US 2004/0058970 A1, US 7176203, US 2010/0216752 A1, US 2008/0064748 A1, US 2002/0103371 A1, US 2008/0064748 A1, US 6395905, US 2004/0235844 A1, US 2004/0006226 A1, EP 1451161 B1, US 2006/0040940 A1, US 2006/0058303 A1, AU 2003/276424, AU 2003/282222, US 2006/0041125 A1, US 7148222, US 6395766, AU 2002/343110, US 6642229, US 2003/0176449 A1, US 2004/0110778 A1, US 2003/0220348 A1, US 2008/0167324 A1, EP 1368342 B1, US 2010/652435 A, US2004006226-A1, US2010130481-A1 and US2008033061-A1. The "inverse agonist of the GABA_{A} receptor that contains α5 subunit" according to the disclosure includes the compounds in the patents above.

The compounds that have been definitely reported in scientific literature with significant inverse agonist effect are shown in Table 1.

**Table 1**

| Name | The literature or patent of synthetic methods | Structure |
|---|---|---|
| MRK-016 | literature (Chambers MS et al., An orally bioavailable, functionally selective inverse agonist at the benzodiazepine site of GABAA alpha5 receptors with cognition enhancing properties. J Med Chem. 2004 Nov 18; 47(24):5829-32.) and patent US 6355638 (Pyrazolo[1,5-d][1,2,4] triazines for enhancing cognition). | 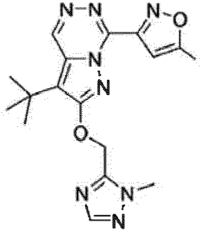 |
| MRK-016-M3 | Jones et al., Pharmacokinetics and metabolism studies on (3-tert-butyl-7-(5-methylisoxazol-3-yl)-2-(1-methyl 1H-,2,4-triazol-5-ylmethoxy) pyrazolo[1,5-d][1,2,4]triazine, a functionally selective GABAA α5 inverse agonist for cognitive dysfunction. Bioorg Med Chem Lett. 2006 Feb 15;16(4):872-5. | 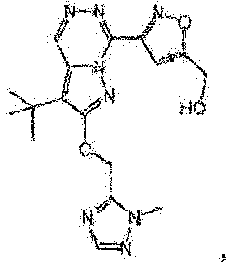 |
| MRK-016-M2 | | 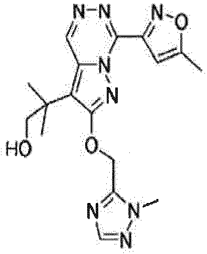 |
| MRK-016-M4 | | 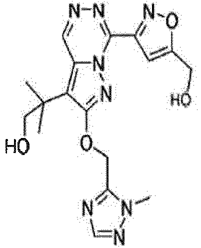 |
| α 5IA | literature (Sternfeld F et al., Selective, orally active gamma-aminobutyric acidA alpha5 receptor inverse agonists as cognition enhancers. J Med Chem. 2004 Apr 22; 47(9):2176-9.) and patent WO 9850385 A1. | 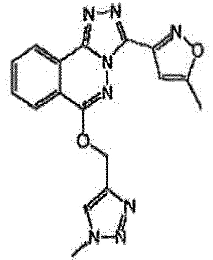 |
| RO4938581 | literature (Knust H et al., The discovery and unique pharmacological profile of RO4938581 and RO4882224 as potent and selective GABA_{A} alpha5 inverse agonists for the treatment of cognitive dysfunction. Bioorg Med Chem letter. 2009 Oct 15;19(20):5940-4) and US patent (US 2006/0084642 A1, US 2007/0082890 A1, etc.). | 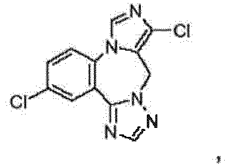 |
| RO4882224 | | 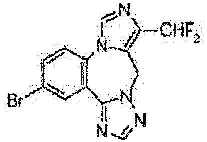 |
| RO | | 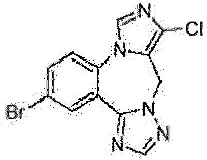 |
| PWZ-029 | Savić MM et al., PWZ-029, a compound with moderate inverse agonist functional selectivity at GABAA receptors containing alpha5 subunits, improves passive, but not active, avoidance learning in rats. Brain Res. 2008 May 7;1208:150-9. | 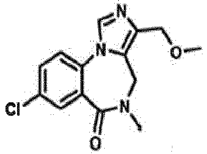 |

The medicines with obvious inverse agonist effect but cannot effectively pass blood brain barrier includes:

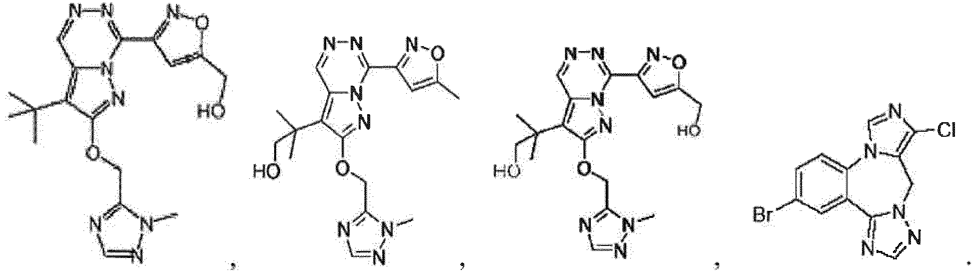

It was reported (Jones et al., Pharmacokinetics and metabolism studies on (3-tert-butyl-7-(5-methylisoxazol-3-yl)-2-(1-methyl 1H-,2,4-triazol-5-ylmethoxy) pyrazolo[1,5-d][1,2,4]triazine, a functionally selective GABAA alpha5 inverse agonist for cognitive dysfunction. Bioorg Med Chem Lett. 2006 Feb 15;16(4):872-5) that MRK016 was metabolized to various structures such as MRK016-M1, MRK016-M2 and MRK016-M3 *in vivo.* MRK016-M3 and MRK016-M2 possessed significant inverse agonist effect of α5-GABA_{A} receptor, but were not capable of binding the α5-GABA_{A} receptor in brain, indicating that the inverse agonist of α5-GABA_{A} receptor that failed to effectively enter blood brain barrier could efficiently treat inflammatory and neuropathic pain. They were the medicines with high safety.

The inventors has also found that compounds 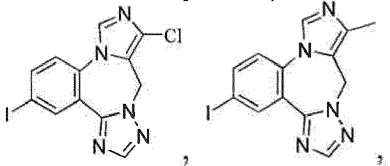 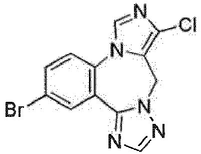 can efficiently suppress inflammatory pain and neuropathic pain. They can barely pass blood brain barrier and will not directly affect central function. These compounds are the medicines that can efficiently treat pain with little central side effect.

This disclosure also includes the isomer, racemate, pharmaceutically acceptable salt, hydrate or precursor of the compounds listed above.

The "pharmaceutically acceptable salt" represents the salt produced by the reaction between the compounds above and inorganic acid, organic acid, alkali metal or alkaline earth metal. These salts include (but not limit to): (1) the salts formed with the following inorganic acids: e.g. hydrochloric acid, hydrobromic acid, hydroiodic acid, sulfuric acid, nitric acid and phosphoric acid; (2) the salts formed with the following organic acids: e.g. acetic acid, lactic acid, citric acid, succinic acid, fumaric acid, gluconic acid, benzoic acid, methane sulfonic acid, ethane sulfonic acid, benzene sulfonic acid, p-toluene sulfonic acid, oxalic acid, succinic acid, tartaric acid, maleic acid or arginine. Other salts include the salts formed with alkali metal or alkaline earth metal (e,g. sodium, potassium, calcium or magnesium), ammonium salts or water-soluble amine salts (e.g. N-methyl glucosamine salt), low-level alkanol ammonium salts as well as other pharmaceutically acceptable amine salts (e.g. methylamine salts, ethylamine salts, propylamine salts, dimethylamide salts, trimethylamide salts, diethylamide salts, triethylamide salts, tert-butylamine salts, ethylenediamine salts, hydroxyethylamine salts, dihydroxyethylamine salts, trihydroxyethylamine salts and the amine salts formed with morpholine, piperazine and lysine, respectively), or other conventional "prodrug" forms. The compounds have one or more asymmetric centers. Therefore, these compounds can exist as racemic mixture, single enantiomer, single diastereoisomer, diastereoisomer mixture or cis or trans isomer.

The "precursor of compound" represents a compound whose precursor can be transformed to a compound with Formula I through metabolic or chemical reactions in patients' body after administrated through proper route, or the salt or solution composed of a compound with Formula I. The precursor of compound includes but not limits to the carboxylic ester, carbonic ester, phosphate ester, nitric acid ester, sulfate, sulfone ester, sulfoxide ester, amino compound, carbamate, azoic compound, phosphamide, glucoside, ether or acetal forms of the compound.

The preparation methods of the compounds of this disclosure are known by those skilled in the art. For example, literature (Chambers MS et al., An orally bioavailable, functionally selective inverse agonist at the benzodiazepine site of GABAA alpha5 receptors with cognition enhancing properties. J Med Chem. 2004 Nov 18; 47(24):5829-32) and patent US 6355638 (Pyrazolo[1,5-d][1,2,4] triazines for enhancing cognition) disclosed the chemical synthesis method of MRK-016. Literature (Jones P et al., Pharmacokinetics and metabolism studies on (3-tert-butyl-7-(5-methylisoxazol-3-yl)-2-(1-methyl-1H-1,2,4-triazol-5-ylmethoxy) pyrazolo[1,5-d][1,2,4]triazine, a functionally selective GABAA alpha5 inverse agonist for cognitive dysfunction. Bioorg Med Chem Lett. 2006 Feb 15;16(4):872-5) disclosed the chemical synthesis method of MRK016-M3. Literature (Sternfeld F et al., Selective, orally active gamma-aminobutyric acidA alpha5 receptor inverse agonists as cognition enhancers. J Med Chem. 2004 Apr 22; 47(9):2176-9) and patent WO 9850385 A1 disclosed the synthetic identification of α5IA. Literature (Atack JR et al., In vitro and in vivo properties of 3-tert-butyl-7-(5-methylisoxazol-3-yl)-2-(1-methyl-1H-1,2,4-triazol-5-ylmethoxy) -pyrazolo[1,5-d]-[1,2,4]triazine (MRK-016), a GABAA receptor alpha5 subtype-selective inverse agonist. J Pharmacol Exp Ther. 2009 Nov;331(2):470-84) disclosed that MRK016, MRK016-M3 and α5IA were all the inverse agonists binding to benzodiazepine binding site and selective for α5-GABA_{A} receptor. The affinity and efficacy between MRK016 (MRK-016) and its major metabolites (M1, M2, M3) and each subtype of GABA_{A} receptor are shown in Table 1.

### Screening method

With the fact that the inverse agonist of α5-GABA_{A} receptor suppresses pain through selectively binding α5-GABA_{A} receptor, the substances that bind α5-GABA_{A} receptor can be screened based on these features. Then, the medicines that are really useful for suppressing pain can be found among these substances.

Therefore, this disclosure provides a method for screening the drugs for preventing, relieving and treating pain, the method includes: (1) providing α5-GABA_{A} receptor system, wherein the α5-GABA_{A} receptor possesses complete GABA_{A} receptor function; (2) administrating candidate substance to the system in step (1) and observing the binding between candidate substance and α5-GABA_{A} receptor; if the candidate substance can bind to the α5-GABA_{A} receptor, then the candidate substance is the a potential drug for preventing, relieving or treating pain. The system that contains the α5 subunit of GABA_{A} receptor can be for example cellular (e.g. dorsal root ganglion cell) system. The cell can be the cell endogenously expressing the α5 subunit of GABA_{A} receptor, or the cell recombinantly expressing the α5 subunit of GABA_{A} receptor. The system of the α5 subunit of GABA_{A} receptor can also be sub-cellular system, solution system, tissue system, organ system or animal system (e.g. animal model, preferably the animal model of non-human mammal, e.g. mouse, rabbit, goat, monkey, etc.). In one aspect of the disclosure, control group can be set during the screening to observe the difference of the binding of the GABA_{A} receptor that contains α5 subunit and the candidate substance more easily. The control group can be the system without administrating the candidate substance.

The candidate substance includes (but not limits to): small molecular compound (the inverse agonist of GABA_{A} receptor), polypeptide, ligand. Preferably, the candidate substance is the substance that cannot pass the blood brain barrier of animals. In one aspect of the disclosure, the method also includes: conducting further cell experiments and/or animal experiments for obtained candidate substance to further select and determine the really useful substance for preventing, relieving or treating pain.

In another aspect, this disclosure also includes the pain-suppressing substance obtained by using the screening method.

The techniques for detecting whether a compound can bind to a receptor or partial subunits of a receptor are known in the art. Many research works have been conducted on detecting whether a compound is the inverse agonist or antagonist of α5-GABA_{A} receptor. For example, in international patent applications WO 92/22652 and WO 94/13799, the combination of α5 β3 and γ2 of GABA_{A} receptor is used to detect the binding between one compound and the receptor. During the process of drug screening, the methods described by Goeders et al. are often used (Goeders NE and Kuhar MJ, Benzodiazepine binding in vivo with [3H]Ro 15-1788. Life Sci, 1985 Jul, 37(4):345-355). There are also many studies on determining the antagonist, agonist or inverse agonistthat binds to α5-GABA_{A} receptor as a ligand, see the methods described in literature (Wafford K A , Whiting P J and Kemp J A , Differences in affinity and efficacy of benzodiazepine receptor ligands on recombinant GABAA receptor subtypes. 1993 Feb, Mol. Pharmacol 43(2)40-244). The methods for identifying whether one drug enter blood brain barrier are relatively broad, it is reported in literature (Jones et al., Pharmacokinetics and metabolism studies on (3-tert-butyl-7-(5-methylisoxazol-3-yl)-2-(1-methyl-1H-1,2,4-triazol-5-ylmethoxy) pyrazolo[1,5-d][1,2,4]triazine, a functionally selective GABAA alpha5 inverse agonist for cognitive dysfunction. Bioorg Med Chem Lett. 2006 Feb 15;16(4):872-5) that it is possible to detect the candidate compound that inhibits the binding of (³H)Ro -15-1788 (a specific inverse agonist labeled with α5-GABA_{A} receptor) in brain. MRK016 can efficiently inhibit of the binding of (³H)Ro-15-1788 in center while MRK016-M3 cannot significantly inhibit of the binding of (³H)Ro-15-1788 in center. The detection can also be carried out by detecting the drugs in various tissues, e.g. detecting the distribution proportion of a drug in brain and plasma to determine whether the drug can effectively enter blood brain barrier.

### Treatment

This invention provides the compounds as claimed for use in the methods for treating pain. The methods can treat acute and chronic pain both safely and efficiently. According to the methods for treating pain provided by this invention, the methods include administrating effective amount of the inverse agonist of α5-GABA_{A} receptor to patients who need such treatment.

This invention is especially applicable for elderly patients suffering pain and people with poor liver function and kidney function. For most elderly people, chronic pain is a common disease, and pain is always thought to be the accompanying symptom of the aging and lesion of middle-aged and elderly people's organs. The pain of elderly people mainly includes the pain from the joints of extremities in bone joint system, back, neck pain, headache or other chronic diseases. The most common manifestation is arthralgia, stiffness and restricted movement, which is medically referred as degenerative osteoarthropathy. Degenerative osteoarthropathy includes articular cartilage degeneration and hyperostosis (bone spur or osteophyte). In the population over 50 year-old, the incidence rate is merely below heart attack. In elderly people over 65 year-old, the incidence rate can be up to 60% ∼ 90%. Osteoarthritis is the most common reason of pain. 80% of the elderly people over 60 year-old suffer osteoarthritis such as arthralgia, sounds during movement, walking disability, morning stiffness, and more severely redness and swelling of joints. However, for elderly patients, there are always other accompanied chronic diseases such as hypertension, diabetes and heart attack. Different sorts of drugs should be administrated simultaneously to treat corresponding diseases. Moreover, the elderly people's tolerance to drugs is low and therefore prone to adverse response.

This invention provides the compounds as defined in claim 1 for use in a method for treating pain, more specifically a method for treating acute pain and chronic pain. The ligands specific to α5-GABA_{A} receptor as defined in claim 1 are used by this invention to treat pain. These drugs can be administrated orally, through subcutaneous injection, through transdermal patch, by intranasal route or through automatic injection device. In this method, the ligand for treating pain is the inverse agonist of α5-GABA_{A} receptor, specific to α5-GABA_{A} receptor. The ligand cannot effectively pass blood brain barrier.

For the methods used to treat pain in this invention, various dosages such as oral and parenteral dosage can be prepared and administrated. Therefore, the compounds involved in this invention can be administrated through injection, i.e. intravenous, intramuscular, intracutaneous, subcutaneous, intraduodenal or intraperitoneal administration. The compounds involved in this invention can also be administrated through inhalation, e.g. nasal inhalation. Furthermore, the compounds in this invention can be transdermally administrated.

### Composition

The compounds of this invention can be used to produce pharmaceutical composition. Medicinal carrier can be solid or liquid. Solid carrier includes powder, tablet, pill, capsule, cachet, suppository and dispersible granules. The solid carrier can be one or several substances, used as diluent, corrigent, adhesive, preservative, tablet disintegrant or packing material. In powder, the carrier is fine dispersed solid. The compound of this invention and fine dispersed active components are in the mixture. In tablet, the compound is mixed with required adhesive carrier with appropriate proportion and suppressed into required shape and size. Preferably, there are 5 to 70% active compound in powder and tablet. Suitable carriers are magnesium carbonate, magnesium stearate, talcum, sugar, lactose, pectin, dextrin, starch, gelatin, carboxymethylcellulose, sodium carboxymethylcellulose, low melting wax and cocoa butter. Likewise, cachet or lozenge, tablet, powder, capsule, pill, cachet and lozenge are also solid dosages that are applicable for oral administration.

For preparing suppository, the mixture of low melting wax emulsion fatty acid glycerides or cocoa butter is molten, stirring to let the active components scattered homogeneously. Then the molten homogeneous mixture is poured into suitable model, letting it cool for solidification. The preparations in the form of solution include solution, suspension and emulsion, e.g. water and aqueous propylene glycol solution. Parenteral injected liquid preparation can be prepared in aqueous polyethylene glycol solution. The aqueous solution suitable for oral administration can be prepared by solving the active components into water and adding appropriate colorant, corrigent, emulsifier and thickener as required.

The aqueous suspension suitable for oral administration can be prepared by scattering fine dispersed the active components into aqueous adhesive substances such as natural or synthetic gum, resin, methylcellulose, carboxymethyl cellulose sodium and other known suspensions.

The sold preparations that transform to liquid preparations for oral administration just before use are also included. These liquid preparations include solution, suspension and emulsion. Besides active components, these preparations also contain colorant, corrigent, stabilizer, buffer agent, synthetic or natural sweetening agent, dispersant, thickener and cosolvent. These pharmaceutical preparations are preferably unit dosage form, in which the preparation is divided into unit dosage forms that contain suitable active components. The unit dosage form can be packaged preparation, with a certain amount of preparation in the package, e.g. packaged tablet, capsule and the powder in bottle or capsule. The unit dosage form can also be capsule, tablet, cachet or lozenge itself, or can be suitable number of any of these powders in the package.

The amount of active components in unit dosage form varies based on particular application and the efficacy of active components, which can be adjusted from 0.01 mg to about 0.1 g. For example, in medical application, 0.1 to about 3 mg of such medicine can be administrated in capsule three times a day. If necessary, the composition can also contain other compatible therapeutic agents.

For therapeutic application, the original dose of the compound used in this invention is 0.001 mg to 10 mg/kg body weight per day. Nevertheless, the dose can vary based on patient's requirement, the severity of the disease to be treated and the compound to be used. Generally speaking, the dose that is below the optimal dose of the compound is used at the beginning, then gradually increasing the dose to achieve the optimal effect. For convenience, total daily dose can be further divided when desired.

The pharmaceutical composition of this invention can also be used in combination with other therapeutic agents or auxiliary agents simultaneously, including but not limiting to morphine and Gabapentin.

Therefore, this invention provides a drug for treating pain. The drug is not only effective, but also without obvious side effect. Another purpose of this invention is to provide a drug with high safety to special patients such as elderly people and patients suffering liver or kidney failure or cardiovascular diseases.

In conjunction with particular examples, this invention is further elaborated hereinafter. It is to be understood that these examples are intended to illustrate the invention. For the experimental methods without particular conditions mentioned in the following examples, routine conditions are generally adopted, as described in J. Sam brook et al., Molecular Cloning: A Laboratory Manual, Science Press, 2002. Alternatively, the conditions recommended by the manufacturer can also be adopted. Unless otherwise specified, all percentage and fraction are calculated by weight.

Unless otherwise defined, the meanings of all the professional and scientific terms used herein are identical to that known by those skilled in the art. Furthermore, the preferred methods and material described herein are just illustrative.

### I. Experimental methods

### (1) Animals

Male SD rats (200-250 g) were used, 6 per group in the cage. All the animals were placed under 12 hours light/cycle (i.e. 12 hours light and 12 hours dark in each day), free to food and water. All the experiments were conducted under the condition that the observers were blind to pharmaceutical treatment.

### (2) Animal model

Spared nerve injury model (SNI): 10% chloral hydrate was injected intraperitoneally to anesthetize rats. The skin on the superior border of posterior limb of the rats was incised. The muscle was longitudinally separated to expose the core of sciatic nerve and the branching below: tibial nerve, common peroneal nerve and sural nerve. The tibial nerve and common peroneal nerve were ligatured and cut and small sural nerve was retained, meanwhile preventing any injury. The muscle and skin were sutured layer by layer, and then antibiotics were injected intraperitoneally for antibacterial purpose. For detailed methods, please refer to Decosterd I and Woolf CJ. Spared nerve injury: an animal model of persistent peripheral neuropathic pain. 2000 Aug; Pain 87(2): 149-58.

Complete Freund's adjuvant (CFA) induced inflammatory pain model: 10% chloral hydrate was injected intraperitoneally (0.3 ml/100 g body weight) for anesthetization, and then 200 µl CFA was subcutaneously injected from the toes and balls of both metapodium. The preparation method of CFA solution: using the CFA solution available from Sigma Inc., Unite States (the saline containing *Mycobacterium tuberculosis*: oil suspension).

Sciatic nerve and dorsal root nerve ligation model: 10% chloral hydrate was injected to rats for anesthetization. The skin on the superior border of posterior limb of the rats was incised. The muscle was longitudinally separated to expose the core of sciatic nerve, and catgut was used for ligation. Dorsal root nerve was ligatured. Routine alcohol disinfection was carried out after shaving the hip and back. 2 cm vertical incisions were made on L3 and L4 vertebrae. The muscle tissue on both sides of the spine was bluntly dissected, and the muscle and skin were expanded by the expanders to expose enough surgical field. Operating forceps were used to damage L3 vertebrae to expose the dorsal root nerve of L5 and L6. Catgut was used for ligation. After 24 hours, reperfusion occurred in rats. Then, dorsal root ganglion, dorsal root nerve, spinal dorsal horn and sciatic nerve were taken respectively.

Pentetrazol induced epilepsy assays: firstly, C5 or 70%PGE400 was injected to rats, and then 100 mg/kg pentetrazol was subcutaneously injected to rats half an hour later. Pentetrazol was dissolved in saline water. Then the convulsive response of rats was observed. Fatholahi standard was adopted to score the convulsive response. Grade 0, no response; Grade 1, rhythmic mouth and face spasm; Grade 2, fluctuating wandering spasm of body; Grade 3, systemic myoclonus and warped hip; Grade 4, body turning to one side; Grade 5, renvers and systemic tetanic convulsion. It took 30 minutes for observation after administration. The highest grade and the latency time from pentetrazol injection to the induction of the highest grade were collected.

Rat tibia cancer pain model is a model reflecting the metastatic cancer pain of clinical patients. 10% chloral hydrate was injected to normal rats for anesthetization. The skin on the superior border of posterior limb of the rats was incised. The muscle was longitudinally separated to expose tibia. 3x10³ MRMT-1 breast cancer cells of homogenous rats were injected in the marrow cavity of tibia. 14 days after surgery, the rats presented obvious hyperalgesia response.

### (3) Immunohistochemistry and western blot experiments

In immunohistochemistry experiment, fixing solution was perfused to the model rats operated in different time to post-fix for 1.5 hours. The tissue that had been immersed in 10% sucrose for more than 24 hours was cut into 14 µm slices by microtome in cryostat, and the slices were kept in -20°C refrigerator. The slices were incubated with antibody (anti-α5-GABA_{A} receptor (Santa Cruz), goat derived, effective dilution ratio 1:100 ∼ 200) overnight and washed by 0.01 M PBS for three times. Then, secondary antibody was used to incubate with the slices for 40 minutes and the slices were washed by 0.01 M PBS for three times in dark. Finally, microscope was used for observation and taking photo. Immuno-fluorescence intensity was quantitatively analyzed by Image-pro.

In western blot experiment, obtained dorsal root ganglion was lysed in RIPA (50 mM Tris (pH7.4), 150 mM NaCl, 1% Triton X-100, 1% sodium deoxycholate, 0.1% SDS as well as sodium orthovanadate, sodium fluoride, EDTA and leupeptin. The proteins were boiled at 100°C for 5 minutes for denaturation. 10-20 µl samples were added into the prepared channels after the protein samples became cool. Then, electrophoresis was conducted at 60 V for 30 minutes. After the samples passed the boundary between the upper layer gel and the lower layer gel, the voltage was adjusted to 90 V until bromophenol blue reached the lower edge of the lower layer gel. After electrophoresis, the SDS gel that contained target bands was cut for transferring. Through electric transferring, the negative charged proteins were transferred to positive charged PVDF membrane. The blocking buffer that contained 5% skim milk powder was used to block the membrane for 1 hour to reduce unspecific binding. Then, the PVDF membrane was immersed in the blocking buffer containing primary antibody and incubated overnight at 4°C or 1-2 hours at room temperature. After that, the PVDF membrane was washed by TBST solution for three times, totally 1 hour. Then, the PVDF membrane was incubated with the secondary antibody that contained horse radish peroxidase for 1 hour at room temperature. After the incubation of secondary antibody, TBST solution was used again to wash for 1 hour. Finally, PVDF membrane was incubated with the substrate of horse radish peroxidase ECL-PLUS for 5 minutes in dark to produce fluorescence signal. Then X-ray film was used to capture the fluorescence signal on PVDF membrane. Developing solution and fixing solution were added on the film for development and fixation.

### (4) Methods for assaying pain

Method for detecting heat hyperalgesia in rats: firstly CFA solution was injected in the thenar of rats. After 24 hours, heat hyperalgesia quantitative detection was adopted to measure the paw withdrawal of rats to heat radiation stimulation. In order to detect the heat pain threshold of rats, the rats were placed in organic glass box with transparent glass plate. Thermal radiometer (BME-410C; CAMS) was used to stimulate the feet of rats. The heat pain threshold is the time from light source reaching the feet of rats to the rats showing foot-lifting response.

Method for detecting mechanical hyperalgesia in rats: firstly CFA solution was injected in the thenar of rats. After 24 hours, mechanical hyperalgesia quantitative detection was adopted to measure the paw withdrawal of rats to normal and harmless mechanical stimulation. Evaluation was conducted according to reference (Chaplan SR et al., Quantitative assessment of tactile allodynia in the rat paw. J Neurosci Methods. 1994 Jul; 53(1):55-63). In this method, the von Frey fibers with different bending force (equivalent to the range of 1-50 g force) were used to apply harmless mechanical stimulation. The rats were placed in organic glass box with metal mesh plate, and the von Frey fibers were applied on the surface of leg thenar. The von Frey fibers were applied vertically to the surface of thenar to generate the force that will trigger slight resistance to the leg. 2-3 seconds were maintained for every leg thenar. If the leg withdrew suddenly, positive records were recorded. The threshold was the mean of the weight that caused 50% paw withdrawal response (5-6 paw withdrawal / 10 stimulations) in overall 10 stimulations. The threshold of mechanical hyperalgesia was measured again at 5-7 days after CFA inflammation to determine whether these animals showed hyperalgesia.

### (5) Drug dissolution

It is noted that only compounds C2, C3 and C4 are according to the invention. Compounds MRK 016, C2, C3, C4, C5, C6, C7, C8 or α5IA (A5I) were dissolved in 70% PGE400 (PGE400:water = 70:30 in volume). For injection dose, MRK016 was 2 mg/kg body weight; A5I was 3 mg/kg body weight; C2, C3, C4, C5, C6, C7 and C8 were 3 mg/kg body weight.

MRK016-M3 was dissolved in 90% PGE400 (PGE400:water = 90:10 in volume). The doses were 10 mg/kg, 3 mg/kg, 1 mg/kg and 0.3mg/kg, respectively for oral administration. Compounds 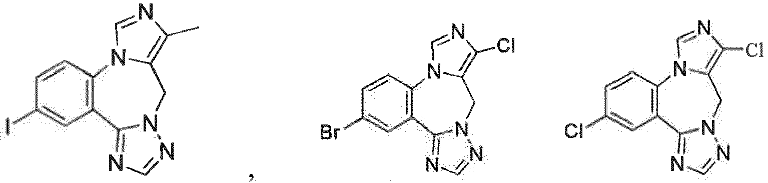 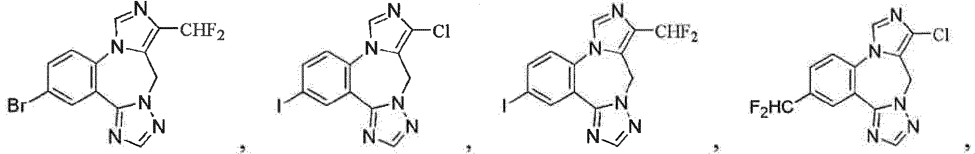 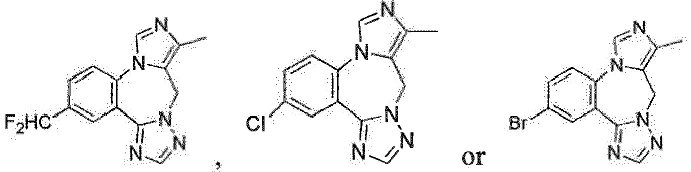 were dissolved in 30% PGE400 (PGE400:water = 30:70 in volume). The dose was 3 mg/kg for injection.

### (6) Knocking-down experiment of α5-GABA_{A} gene

According to the method described in EX 500, siRNA was prepared as injectable solution. The rats were anesthetized and then the siRNA solution was injected into spinal cord at L5 or L6. 3 days after the injection of siRNA, the dorsal root ganglion of L5 or L6 was taken to detect the expression level of the α5 subunit protein of GABA_{A} receptor.

The siRNA sequence specific to the mRNA of α5-GABA_{A} receptor was: 5'GGUGCGAACAGACAUCUAUTT 3'(SEQ ID NO: 1). The control siRNA sequence was: 5'UUCUCCGAACGUGUCACGUTT 3' (SEQ ID NO: 2).

### (7) Data statistics

The method for behavioral data statistics was unpaired t-test. *, p<0.05, represents significant difference.

### II. Examples

### Example 1. The expression level of the α5 subunit of GABA_{A} receptor was elevated in SNI model

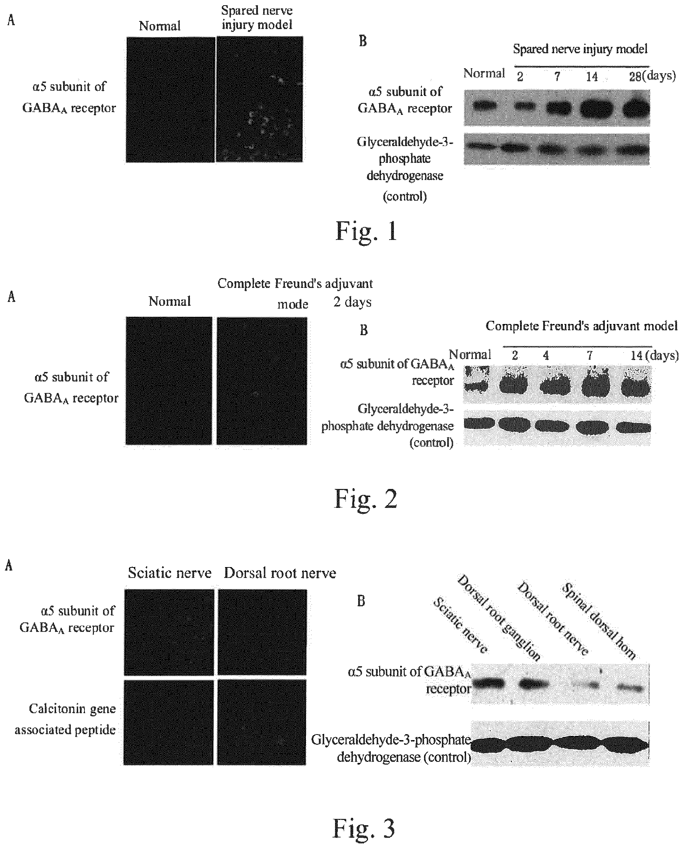

In SNI model, the expression level of GABA_{A} receptor α5 subunit was measured by immunohistochemistry experiment and western blot experiment. The results were shown in Fig. 1.

The immunohistochemistry experiment indicated that SNI model induced the elevation of the expression level of the α5 subunit of GABA_{A} receptor. 14 days after SNI model, the expression level of the α5 subunit of GABA_{A} receptor increased. The dorsal root ganglion slices of normal rats were used as control (normal), as shown in Fig. 1A. The results indicated that the expression level of the α5 subunit of GABA_{A} receptor increased in dorsal root ganglion. Interestingly, the elevation of the fluorescence signal of the α5 subunit protein of GABA_{A} receptor was more obvious in small neurons.

The western blot experiment indicated that total protein amount of the α5 subunit of GABA_{A} receptor increased in SNI model, as shown in Fig. 1B.

### Example 2. The expression level of the α5 subunit of GABA_{A} receptor was elevated in inflammatory pain model

In CFA model, the expression level of the α5 subunit of GABA_{A} receptor was measured by immunohistochemistry experiment and western blot experiment. The results were shown as Fig. 2.

The immunohistochemistry experiment indicated that the expression level of the α5 subunit of GABA_{A} receptor in dorsal root ganglion was elevated at 2 days after the injection of CFA in the CFA induced inflammatory pain model, as shown in Fig. 2A.

In immunohistochemistry experiment, it was found that the expression level of the α5 subunit of GABA_{A} receptor increased in the small cells in dorsal root ganglion. The statistics showed that the proportion of the cells expressing the α5 subunit of GABA_{A} receptor in dorsal root ganglion increased. Western blot experiment indicated that the protein amount of the α5 subunit of GABA_{A} receptor in inflammatory pain model was significantly elevated (Fig. 2B). Glyceraldehyde-3-phosphate dehydrogenase (GAPDH) was used as positive control.

### Example 3. The α5 subunit protein of GABA_{A} receptor was expressed in dorsal root ganglion and transported to sciatic nerve

In sciatic nerve and dorsal root nerve ligation model, the protein amount of the α5 subunit of GABA_{A} receptor was observed, as shown in Fig. 3.

Fig. 3A indicated that the α5 subunit protein of GABA_{A} receptor was transported to sciatic nerve, but barely transported to dorsal root nerve. CGRP (calcitonin gene associated peptide) is a polypeptide transported along sciatic nerve and dorsal root nerve. It was used as positive control. Western blot experiment indicated that protein amount of the α5 subunit of GABA_{A} receptor in sciatic nerve, dorsal root ganglion cell body, dorsal root nerve bundle and spinal dorsal horn was significantly elevated.

Fig. 3B indicated that protein amount of the α5 subunit of GABA_{A} receptor in sciatic nerve was obviously higher than that in dorsal root nerve, and the protein amount of the α5 subunit of GABA_{A} receptor in spinal cord was much less. Glyceraldehyde-3-phosphate dehydrogenase (GAPDH) was used as positive control.

### Example 4. The specific inverse agonist of α5-GABA_{A} receptor efficiently suppressed neuropathic pain (reference)

SNI model mimics the neuropathic pain that is related with nerve injury. The injury can be caused directly by trauma and crush, or indirectly by a series of diseases. The diseases include infection, cancer, metabolism diseases, toxin, nutrition deficiency, immunity dysfunction and musculoskeletal changes. In current clinical treatment, neuropathic pain is one of the most difficult pains, seriously affecting patients' life quality. Currently, gabapentin is the first-line drug for treating neuropathic pain. However, gabapentin may cause some side effects such as somnolence, dizziness, unsteady walking and feelings of fatigue.

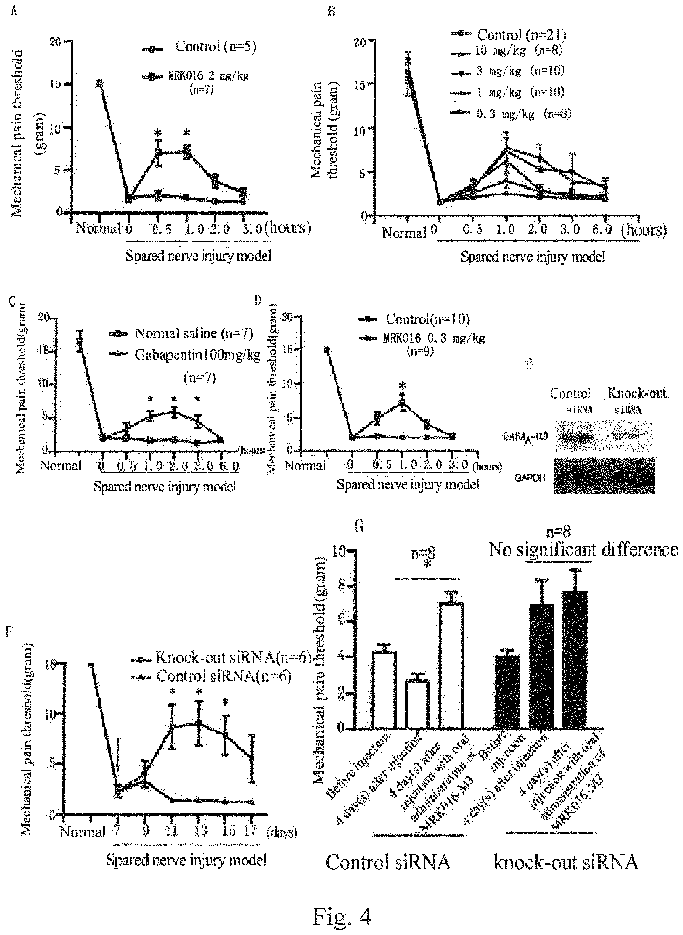

The inventors found that compound MRK016 and its metabolite MRK016-M3 efficiently suppressed pain. At 7 days after SNI model, subcutaneous injection of MRK016 efficiently suppressed neuropathic pain. The results showed that the pain was obviously suppressed at 1 hour after administration, as shown in Fig. 4A.

The inventors detected whether MRK016-M3 was capable of suppressing neuropathic pain. Through dose-dependent experiment, it was found that MRK016-M3 efficiently suppressed neuropathic pain, as shown in Fig. 4B. In this experiment, rats that were 10 to 20 days after the establishment of SNI model were used as experimental animals. The results showed that mechanical pain was significantly suppressed at the dose of 10 mg/kg, 3 mg/kg and 1 mg/kg even at 1 hour after oral administration.

Meanwhile, the inventors detected the effect of gabapentin on treating neuropathic pain. Rats that were 11 days after the establishment of SNI model were used as experimental animals. It was found that gabapentin efficiently suppressed neuropathic pain, as shown in Fig. 4C. However, the dose of MRK016-M3 was much less than the dose of gabapentin, with similar effects of treating pain.

In order to further confirm that MRK016-M3 functioned through peripheral nerve, the inventors used rats that were 10 days after the establishment of SNI model as experimental animals for topical administration. 100 microliter MRK016-M3 (1 mg/ml) was injected to the feet of rats, and the results indicated that neuropathic pain was still efficiently suppressed, as shown in Fig. 4D.

In order to verify whether the drug functioned through binding α5-GABA_{A} receptor, the function of α5-GABA_{A} receptor in SNI model was detected by gene knocking-down experiment. Through intrathecal injection of the siRNA specific to the mRNA of the α5 subunit of GABA_{A} receptor, the inventors found by statistics that the method significantly knocked-out the expression of the α5 subunit of GABA_{A} receptor in dorsal root ganglion, as shown in Fig. 4E.

According to western blot experiment, it was found that the injection of specific siRNA significantly reduced the expression of the α5 subunit protein of GABA_{A} receptor in dorsal root ganglion. For control siRNA, there was no obvious change on the α5 subunit protein of GABA_{A} receptor. Glyceraldehyde-3-phosphate dehydrogenase (GAPDH) was used as positive control. In SNI model, it was found that intrathecal injection of the siRNA specific to the α5 subunit protein of GABA_{A} receptor significantly elevated pain threshold. For unspecific siRNA, there was no obvious effect on pain, as shown in Fig. 4F. In this experiment, at 7 days after the establishment of SNI model, the inventors injected the siRNA specific to the α5 subunit protein of GABA_{A} receptor to the spinal cord of rats, and then the pain threshold of rats were detected at 9 days, 11 days, 13 days, 15 days and 17 days after the establishment of SNI model. In order to further verify that MRK016-M3 suppressed chronic pain through α5-GABA_{A} receptor, the pain threshold of rats was detected at 4 days after the establishment of SNI model. Then control siRNA and the siRNA specific to the α5 subunit of GABA_{A} receptor were injected. The effect of MRK106-M3 on the pain threshold of rats was detected 4 days later. The inventors' results indicated that MRK016-M3 (oral administration, 3 mg/kg) significantly suppressed neuropathic pain in the rats injected with control siRNA; while there was no obvious effect of MRK016-M3 (oral administration, 3 mg/kg) in the rats injected with the siRNA specific to the α5 subunit of GABA_{A} receptor ,as shown in Fig. 4G.

According to the mechanism above, those skilled in the art can understand that the substances that can block the biological function of GABA through α5-GABA_{A} receptor are all potential substances for preventing and treating pain.

It was reported in literature (Jones P et al., Pharmacokinetics and metabolism studies on (3-tert-butyl-7-(5-methylisoxazol-3-yl)-2-(1-methyl-1H-1,2,4-triazol-5-ylmethoxy) pyrazolo [1,5-d] [1,2,4]triazine, a functionally selective GABAA alpha5 inverse agonist for cognitive dysfunction. Bioorg Med Chem Lett. 2006 Feb 15;16(4):872-5) that MRK016 and its metabolite MRK016-M3 possessed similar inverse agonist effect to α5-GABA_{A} receptor, but MRK016 was focused in the brain while MRK016-M3 could hardly enter into the brain to bind with the α5-GABA_{A} receptor, suggesting that MRK016-M3 cannot binding the α5-GABA_{A} receptor expressed in center and leading to side effects in center system. Instead, it can suppress pain response efficiently and is a potential clinical analgesic drug.

Meanwhile, the experiment also proved that the inverse agonist of the α5-GABA_{A} receptor functioned in peripheral nerve could be used to treat neuropathic pain. The conclusions above indicated that the specific inverse agonist that bound α5-GABA_{A} receptor but did not pass blood brain barrier could efficiently suppress neuropathic pain, providing a new screening method for potential clinical analgesic drugs.

### Example 5. Treatment of inflammatory pain by the inverse agonist of α5-GABA_{A} receptor (reference)

Generally, inflammatory pain is the pain caused by the inflammation induced by the lesion of tissue or organ, including acute pain and chronic pain. In animal experiments, tissue injury pathological pain model is the model similar with clinical inflammatory pain, wherein CFA induced inflammatory pain model reflects most of the indictors in clinical chronic inflammatory pain. In this model, inflammatory response occurs on the skin of injection site at 2-3 hours after injection, with the peak at 6-24 hours. Hyperalgesia and edema can last for 1-2 weeks (Ladarola et al., Differential activation of spinal cord dynorphin and enkephalin neurons during hyperalgesia: evidence using cDNA hybridization." Brain Res. 1988 , vol.455 , No.2 , pp:205-12). The feature of inflammatory pain in this model is significant and the duration time is long. The inventors found that MRK016 and its metabolite MRK016-M3 efficiently suppressed inflammatory pain.

Spinal injection of the siRNA specific to the α5 subunit of GABA_{A} receptor could inhibit the expression of the α5 subunit of GABA_{A} receptor in dorsal root ganglion and inhibit the inflammatory pain efficiently, as shown in Fig. 5A and B. In this experiment, the inventors firstly detected the heat pain threshold and mechanical pain threshold of rats, and then the siRNA specific to the α5 subunit of GABA_{A} receptor was injected at the same day. At the 2^{nd} day, 100 microliter CFA was injected to the right foot of rats. Then the heat pain threshold and mechanical pain threshold of rats were detected every day. The results showed that the inhibition of the expression of the α5 subunit of GABA_{A} receptor in dorsal root ganglion significantly suppressed inflammatory pain.

According to the results above, those skilled in the art can understand that the substances that can block the biological function of GABA through α5-GABA_{A} receptor are all potential substances for preventing and treating pain.

Intraperitoneal injection of MRK016 (2 mg/kg body weight) could efficiently suppress inflammatory pain. In this experiment, at 7 days after the establishment of inflammatory pain model, a period of time when rats were sensitive to mechanical pain, the inventors intraperitoneally injected MRK016 (2 mg/kg body weight). It was found that the substance efficiently suppressed mechanical pain (Fig. 5C). At 3 days after the establishment of inflammatory pain model, a period of time when rats were sensitive to heat pain, MRK016 (2 mg/kg body weight) was injected intraperitoneally. It was found that the compound efficiently suppressed heat pain (Fig. 5D). The results indicated that MRK016 suppressed inflammatory pain at 1 hour after administration.

Oral administration of MRK016-M3 also efficiently suppressed inflammatory pain. The inventors detected heat pain at 3 days after the establishment of inflammatory pain model and detected mechanical pain at 7 days after the establishment of inflammatory pain model, respectively. The results indicated that MRK016-M3 could significantly suppress inflammatory pain at 1 hour after administration, as shown in Fig. 5E and F.

Meanwhile, the experiment also proved that the inverse agonist of the α5-GABA_{A} receptor functioned in peripheral nerve could be used to treat inflammatory pain. The conclusions above indicated that the specific inverse agonist that bound α5-GABA_{A} receptor but did not pass blood brain barrier could efficiently suppress inflammatory pain, providing a new screening method for potential clinical analgesic drugs.

### Example 6. Treatment of neuropathic pain and inflammatory pain by the inverse agonist of α5-GABA_{A} receptor A5I (reference)

a5IA (A5I) is an inverse agonist specific to α5-GABA_{A} receptor developed by Merck Sharp & Dohme (Merck). In phase II clinical trials, certain toxic problems were found due to the water solubility of the drug.

It was reported in literature (Munro G et al., A question of blance-positive versus negative allosteric modulation of GABAA receptor subtypes as drive of analgesi efficacy in rat model of inflammatory and neuropathic pain. Neuropharmacology. 2011 Jul-Aug;61(1-2):121-32) that there was no effect of this compound on treating pain. Here, the inventors detected the analgesic effect of A5I in SNI and inflammatory pain model. The data showed that intraperitoneal injection of A5I (3 mg/kg body weight) could efficiently treat neuropathic pain, as shown in Fig. 6A. In this experiment, at 14 days after SNI model, A5I (3 mg/kg) was injected subcutaneously, and the nociceptive threshold of rats were measured at 0.5 hour, 1 hour, 2 hours, 3 hours and 24 hours after administration. The results indicated that the compound efficiently suppressed neuropathic pain.

Continuous administration of A5I did not produce tolerance. In this experiment, at 14 days after the establishment of SNI model, the drug was administrated continuously for 6 days, and the nociceptive threshold of rats were measured at 1-3 hours after administration respectively. It was found that pain was still efficiently treated even after continuous administration for 6 days, as shown in Fig. 6B.

In CFA model, the inventors found that intraperitoneal injection of A5I (3 mg/kg body weight) also could efficiently inhibit inflammatory pain, as shown in Fig. 6C and D. In this experiment, at 3 days after CFA injection, the effect of A5I on heat pain was detected. The pain threshold was measured at 0.5 hour, 1.0 hour, 2.0 hours and 3.0 hours after administration. At 7 days after CFA injection, the effect of A5I on mechanical pain was detected. The mechanical pain threshold was measured at 0.5 hour, 1.0 hour, 2.0 hours and 24 hours after administration.

### Example 7. Method of screening drugs (reference) (1) Cellular level screening

Different subunits of GABA_{A} receptor were expressed in cell lines, including human kidney epithelial cell line, 293 cell line or L(tk-) cell line. The cells were cultured in medium, and such cells were used as the cellular model for screening drugs to suppress pain. α subunit, β subunit and γ subunit are indispensable for one complete functional GABA_{A} receptor.

In this example, the inventors established the following cellular models:
(a) simultaneously expressing α5 subunit (for the protein sequence, see GenBank accession number NM_001165037.1), β3 subunit (for the protein sequence, see GenBank accession number NM_021912.4) and γ2 subunit (for the protein sequence, see GenBank accession number NM_198904.2) in 293 cell line, forming the GABA_{A} receptor containing α5 subunit;
(b) simultaneously expressing α1 subunit (GenBank accession number: NM_001127648.1), β3 subunit and γ2 subunit in 293 cell line, forming the GABA_{A} receptor containing α1 subunit;
(c) simultaneously expressing α2 subunit (for the protein sequence, see GenBank accession number NM_000813.2), β3 subunit and γ2 subunit in 293 cell line, forming the GABA_{A} receptor containing α2 subunit;
(d) simultaneously expressing α3 subunit (for the protein sequence, see GenBank accession number NM_000808.3), β3 subunit and γ2 subunit in 293 cell line, forming the GABA_{A} receptor containing α3 subunit;
Each of the (a) to (d) above could form a GABA_{A} receptor with complete function.

Then, the cellular membrane was extracted and the protein concentration was measured. The competitive binding of the substances to be measured and radiolabelled compounds (e.g. 1 nM [³H]Ro-0151788) were detected respectively to calculate the Ki value of the substances to be measured, see the method described in literature (Ballard TM et al., RO4938581, a novel cognitive enhancer acting at GABA_{A} alpha5 subunit-containing receptors. Psychopharmacology (Berl). 2009 Jan; 202(1-3): 207-23). If the binding inhibition constant Ki (nM) of α5-GABA_{A} receptor with the candidate substances in experimental group is less than the Ki of the receptor containing other α subunit (α1 subunit, α2 subunit or α3 subunit), then the candidate substance is a potential drugs for preventing, relieving or treating pain. Many methods can be used by those skilled in the art to evaluate the binding capacity of the ligand of α5-GABA_{A} receptor to the α5-GABA_{A} receptor, including but not limiting to the method above.

The inventors used electrophysiological methods to measure the inverse agonist efficacy of the substances to be measured, see the method described in literature (Ballard TM et al., RO4938581, a novel cognitive enhancer acting at GABA_{A} alpha5 subunit-containing receptors. Psychopharmacology (Berl). 2009 Jan; 202 (1-3): 207-23). If the substance to be measured can significantly inhibit the current induced by GABA through α5-GABA_{A} receptor, but meanwhile cannot significantly inhibit the current induced by GABA through the GABA_{A} receptor that contains α1 or α2 or α3, then the candidate substance is a potential drug for preventing, relieving and treating pain. Many methods can be used by those skilled in the art to evaluate the binding capacity of the ligand of α5-GABA_{A} receptor to the α5-GABA_{A} receptor, including but not limiting to the method above.

Through *in vitro* blood-brain barrier model (BBBM), whether the substance to be measured could effectively pass blood brain barrier was detected. In this experiment, brain microvessl endothelial cells were co-cultivated with astrocyte, and then whether the substance to be measured could effectively pass cellular membrane was detected. For detailed method, please refer to related literature (Culot M et al., An in vitro blood-brain barrier model for high throughput (HTS) toxicological screening. Toxicol In Vitro. 2008 Apr; 22(3):799-811).

MRK016, MRK016-M3 and α5IA were chosen as candidate substances for measurement. The results were shown in Table 2. The affinity between MRK016, MRK016-M3 and α5IA and various subtypes of GABA_{A} receptor were similar. But the inverse agonist efficacy to α5-GABA_{A} receptor is much higher than that to the GABA_{A} receptor containing α1 or α2 or α3. Thus they are useful substances for suppressing pain in mammal. Preferably, MRK016-M3 cannot significantly pass blood brain barrier. Theoretically it is an effective analgesic drug with no side effect.

Therefore, if the binding constant Ki (nM) of the α5 subunit of γ-aminobutyric acid A with the candidate substances in experimental group is less than the Ki of other subunits (α1 subunit, α2 subunit or α3 subunit), then the candidate substance to be measured is a potential drugs for preventing, relieving or treating pain. Alternatively, if the candidate substance to be measured can significantly inhibit the current induced by GABA through the GABA_{A} receptor containing α5 subunit, but meanwhile cannot significantly inhibit the current induced by GABA through the GABA_{A} receptor that contains α1 or α2 or α3, then the candidate substance is a potential drug for preventing, relieving or treating pain or the combination thereof. The results above indicate that the candidate substance is a potential drug for preventing, relieving or treating pain.

**Table 2**

| Name | Indicator | Human recombinant GABA A subtypes (the α subunit contained) | | | |
|---|---|---|---|---|---|
| | | α1 | α2 | α3 | α5 |
| MRK016 | Affinity, nM | 0.83 | 0.85 | 0.77 | 1.36 |
| | Inverse agonist efficacy, % | -16 | 6 | -9 | -55 |
| MRK016-M2 | Affinity, nM | 26 | N.D. | 9.8 | 9.4 |
| | Inverse agonist efficacy, % | -41 | -23 | -32 | -40 |
| MRK016-M3 | Affinity, nM | 1.5 | N.D. | 0.69 | 1.1 |
| | Inverse agonist efficacy, % | -11 | -1 | -15 | -52 |
| α5IA | Affinity, nM | 0.88 | 0.58 | 0.61 | 0.66 |
| | Inverse agonist efficacy, % | -4 | 12 | 4 | -29 |

Inverse agonist efficacy represents the inhibition percentage of the candidate substance on the current induced by GABA through α5-GABA_{A} receptor = (the current intensity induced by GABA through α5-GABA_{A} receptor with the existence of candidate substance - the current intensity induced by GABA through α5-GABA_{A} receptor without the existence of candidate substance) / the current intensity induced by GABA through α5-GABA_{A} receptor without the existence of candidate substance.

### (2) Method of drug screening using animals

The candidate substances were administrated to animals by intraperitoneal injection (or oral administration). After proper time of treatment, the distribution of the candidate substance in animals was measured. Two methods could be used to detect the binding efficacy between the compound and central α5-GABA_{A} receptor.

The first method was to measure the pharmaceutical distribution of the substance in animal tissues. In this method, measurement was conducted to determine whether the substance to be measured could compete with or inhibit the binding of radiolabelled substance such as (³H)Ro-15-1788 in hippocampus. The inhibition efficacy was no more than 60%. Certain dose of candidate substance and (³H)Ro-15-1788 were administrated to experimental animals, and then whether the candidate substance could inhibit the distribution of the ligand specific to known α5-GABA_{A} receptor in center was detected. If the distribution of (³H)Ro-15-1788 in center inhibited by the candidate substance is less than 60%, then the candidate substance is a potential drug for preventing, relieving and treating pain with fewer side effects. The results indicated that 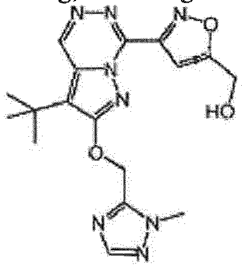 (MRK016-M3) could not significantly inhibit the binding of (³H)Ro-15-1788 in center efficiently. The inhibition rate was about 12% (Jones P et al., Pharmacokinetics and metabolism studies on (3-tert-butyl-7-(5-methylisoxazol-3-yl)-2-(1-methyl-1H-1,2,4 -triazol-5-ylmethoxy) pyrazolo [1,5-d][1,2,4]triazine, a functionally selective GABAA alpha5 inverse agonist for cognitive dysfunction. Bioorg Med Chem Lett. 2006 Feb 15; 16(4):872-5).

The second method was to measure the tissue distribution of the substance to be measured in animal tissues. Firstly the candidate substance was administrated to animals by injection (or oral administration). The brain tissue and plasma tissue were extracted 0.25-2.0 hours after injection. liquid Chromatograph Mass SpectrometerL-MS was used to measure the concentration of the drug. If the brain/plasma ratio of the compound is less than 0.5, then the candidate substance is a potential drug for treating pain with fewer side effects. The results indicated that brain/plasma ratio of 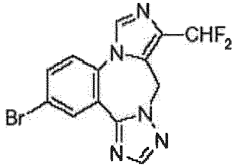 (slightly passing blood brain barrier) and 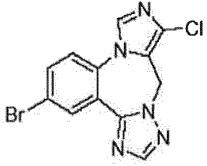 (more slightly passing blood brain barrier) were less than 0.5, showing that these two compounds were potential drugs for treating pain with fewer side effects.

### Example 8. The preparation of (3-(3-tert-butyl -2-((2-methyl-1,2,4-triazol-3-yl)methoxy)pyrazolo[1,5-d][1,2,4]triazin-7-yl)isoxazol-5 -yl)methanol and its analogs (only compounds C2, C3 and C4 are according to the invention, the other compounds are for reference)

### Intermediate 1: 4-tert-butyl-3-formyl-1H-pyrazol-5-yl 4-methylbenzenesulfonate

### (a) 3-tert-butyl-4-hydroxyfuran-2(5H)-one

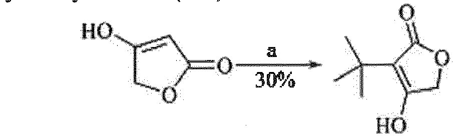

4-hydroxyfuran-2(5H)-one (4.0 g, 40 mmol) and 2-methyl-2-propyl alcohol (3.0 g, 40 mmol) were mixed and stirred for 15 minutes at 45°C. Then the mixture was rapidly treated by concentrated sulfuric acid at 45°C and stirred for 48 hours at 45°C. Then the mixture was diluted by water (10 mL) and extracted by diethyl ether (10 mL x 3). The organic layer was desiccated by magnesium sulfate (MgSO₄). The solvent was evaporated and the residue was conducted for chromatography on silica gel. Through the application of hexane: ethyl acetate = 20: 1 to 10: 1, 1.85 g 3-tert-butyl-4-hydroxyfuran-2(5H)-one was obtained. The substance was a white solid.

### (b) 4-tert-butl-3-hydroxymethyl-1H-pyrazol-5-ol

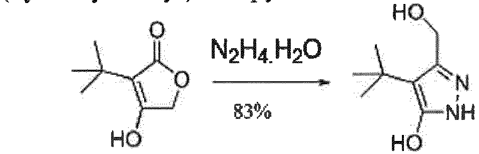

3-tert-butyl-4-hydroxyfuran-2(5H)-one (2.2 g, 14 mmol) was dissolved in ethanol (22 mL) at room temperature. Then hydrazine hydrate (4.1 g, 70 mmol) was added, stirring for 72 hours at 78°C. The reacting solution was evaporated and water (20 mL) was used for washing. Then ethyl acetate was used for extraction (20 mL x 3) and the organic layer was desiccated by MgSO₄. 2 g product (4-tert-butyl-3-(hydroxymethyl)-1H-pyrazol-5-ol) was obtained after low pressure evaporation. The product was a white solid.

### (c) 4-tert-butyl-3-(hydroxymethyl)-1H-pyrazol-5-yl 4-methylbenzenesulfonate

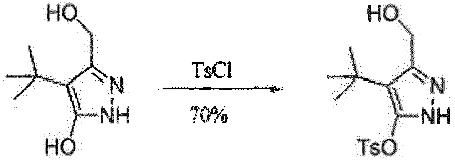

4-tert-butyl-3-(hydroxymethyl)-1H-pyrazol-5-ol (2.0 g, 12 mmol) was stirred in DCM (40 mL) at room temperature. Et3N (1.55 g, 2.12 mL) was added dropwise, stirring for 20 hours at room temperature. The mixture was washed by brine (20 mL) and extracted by DCM (10 mL x 3). The organic layer was desiccated by magnesium sulfate (MgSO₄). The residue was conducted for chromatography on silica gel. With DCM : MeOH = 30:1, 2.7 g product (4-tert-butyl-3-(hydroxymethyl)-1H-pyrazol-5-yl 4-methylbenzenesulfonate) was obtained, which was a yellow solid.

### 4-tert-butyl-3-formyl-1H-pyrazol-5-yl 4-methylbenzenesulfonate

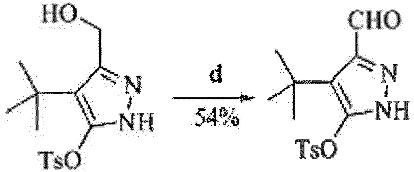

4-tert-butyl-3-(hydroxymethyl)-1H-pyrazol-5-yl 4-methylbenzenesulfonate (100 mg, 0.31 mmol) was dissolved in CHCl₃ (2 mL) at room temperature. Then MnO₂ (70 mg, 0.8 mmol) was added, stirring for 48 hours at 70°C. MnO2 (34 mg, 0.4 mmol) was added again, stirring for 6 hours at 70°C. After cooling to room temperature, the mixture was filtered. Then the solvent was evaporated and the residue was conducted for chromatography on silica gel. With DCM : MeOH = 50:1 for elution, 30 mg product (4-tert-butyl-3-formyl-1H-pyrazol-5-yl 4-methylbenzenesulfonate) was obtained, which was a yellow solid.

### Intermediate 2: 5-(chloromethyl)-1-methyl-1H-1,2,4-triazole

### (a) 2-methyl-2H-1,2,4-triazol-3-yl)methanol

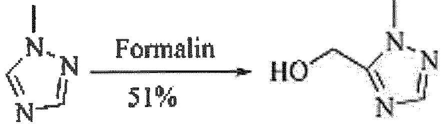

1-methyl-1H-1,2,4-triazol (200 mg, 1.8 mmol) and formalin (1 mL) were mixed in a closed container overnight at 135°C. After cooling to room temperature, the solvent was evaporated in vacuum. The residue was conducted for chromatography on silica gel. With DCM : MeOH = 20:1 for elution, 140 mg product (2-methyl-2H-1,2,4-triazol-3-yl)methanol) was obtained, which was a white solid.

### (b) 5-(chloromethyl)-1-methyl-1H-1,2,4-triazole

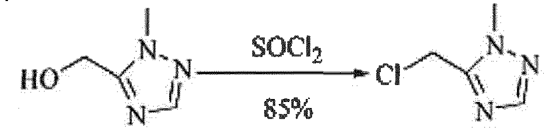

2-methyl-2H-1,2,4-triazol-3-yl)methanol (300 mg, 2.7 mmol) was mixed with SOCl₂ (5 mL) on ice under the condition of inert gas argon. The mixture was heated for 1 hour at 80°C. The solvent was evaporated in vacuum and the residue was grinded with tert-butyl methyl ether (TBME) and filtered. 380 mg product (5-(chloromethyl)-1-methyl-1H-1,2,4- triazole) was obtained, which was a white solid.

### Intermediate 3:

### (a) ethyl 5-(hydroxymethyl) wasoxazole-3-carboxylate

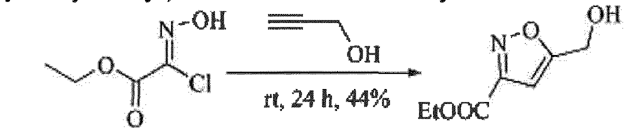

Chlorooximinoethyl acetate (8.0 g, 53 mmol) was dissolved in chloroform (80 mL), and propargyl alcohol (9.0 g, 158 mmol) was added at room temperature. After 15 minutes, triethylamine (10.7 g, 105.6 mmol) was added, stirring for 48 hours at 45°C. The reacting solution was evaporated and the residue was conducted for chromatography on silica gel. With Hexane : EtOAc = 3:1 for elution, 4 g product (ethyl 5-(hydroxymethyl) wasoxazole-3- carboxylate) was obtained, which was a white oily matter.

### (b) 5-(hydroxymethyl)wasoxazole-3-carbohydrazide

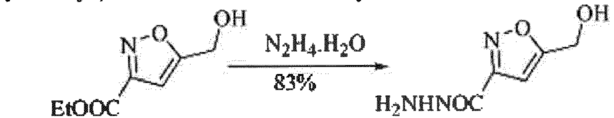

Ethyl 5-(hydroxymethyl) wasoxazole-3- carboxylate (4.3 g, 25 mmol) was dissolved in ethanol (43 mL), and hydrazine hydrate (3.7 g, 63 mmol) was added at room temperature, stirring for 3 hours at room temperature. The reacting solution was filtered and washed by ethanol (10 mL). After desiccated by model pump, 3.4 g product (5-(hydroxymethyl)wasoxazole-3-carbohydrazide) was obtained, which was a white solid.

### (c) 5-tert-butyldimethylsilyloxy)methyl)isoxazole-3-carbohydrazide

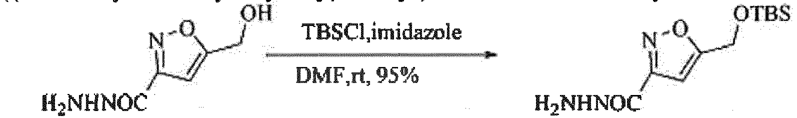

5-(hydroxymethyl)wasoxazole-3-carbohydrazide (550 mg, 3.5 mmol) was dissolved in dry DMF (15 mL), and imidazole (380 mg, 5.6 mmol) was added. Then t-BuMe2SiCl (630 mg, 4.2 mmol) was dissolved in DMF and dropwise added to the solution above at the condition of -10°C. The reacting solution was mixed overnight at room temperature. The reaction was terminated by cold water and ethyl acetate, respectively. The water layer was extracted by using ethyl acetate (50 mL x 2). The organic layer was washed by brine (20 mL x 2) and desiccated by sodium sulfate. After filtration and evaporation, 0.9 g 5-((tert-butyldimethylsilyloxy)methyl)isoxazole-3-carbohydrazide was obtained, which was a faint yellow solid.

### Synthetic step 1

### (a) 3-tert-butyl-7-(5-((tert-butyldimethylsilyloxy)methyl)isoxazol-3-yl)pyrazolo[1,5-d] [1,2,4]triazin-2-yl 4-methylbenzenesulfonate

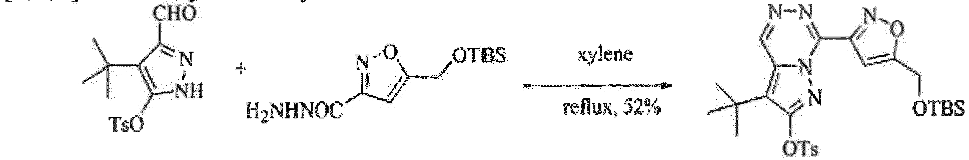

Under the condition of argon, 5-((tert- butyldimethylsilyloxy)methyl)isoxazole-3-carbohydrazide (850 mg, 3.1 mmol) and 4-tert-butyl-3-formyl-1H-pyrazol-5-yl 4-methylbenzenesulfonate (1.1 g, 2.8 mmol) were refluxed in xylene (20 mL) for 72 hours. The residue was obtained after evaporation and grinded with hot methanol. The solid was collected by filtration. 750 mg target intermediate, a brown solid, was obtained after vacuum desiccation. The mother liquor was concentrated and the residue was conducted for chromatography on silica gel. With DCM : MeOH = 200:1 for elution, relatively pure 200 mg intermediate was obtained, which was a brown foamy substance.

### (b) 3-tert-butyl-7-(5-(hydroxymethyl)isoxazol-3-yl)pyrazolo[1,5-d][1,2,4] triazin-2-ol

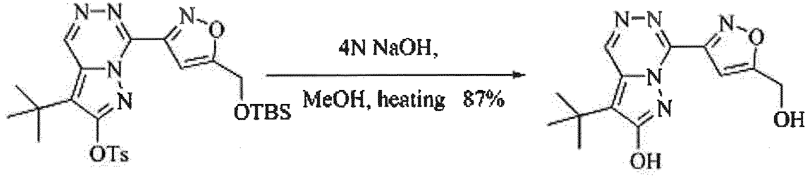

3-tert-butyl-7-(5-((tert-butyldimethylsilyloxy)methyl)isoxazol-3-yl)pyrazolo[1,5-d] [1,2,4]triazin-2-yl-4-methylbenzenesulfonate (800 mg, 1.4 mmol) was dissolved in methanol (20 mL), and 4 N aq. NaOH (1 mL) was added. The reacting solution was mixed under the condition of argon for 2 hours at 60°C and cooled to room temperature. 2 N aq. HCl (2.5 mL) was added. The mixture was filtered and the filtrate was evaporated. The residue was conducted for chromatography on silica gel. With DCM : MeOH = 20:1 for elution, the product was obtained. The mixture was washed by brine twice and desiccated by sodium sulfate. 360 mg product was obtained after filtration, which could be used directly for the next step.

### (c) (3-(3-tert-butyl -2- ((2-methyl-2H-1,2,4-triazol-3-yl)methoxy)pyrazolo [1,5-d][1,2,4]triazin-7-yl)isoxazol-5-yl)methanol

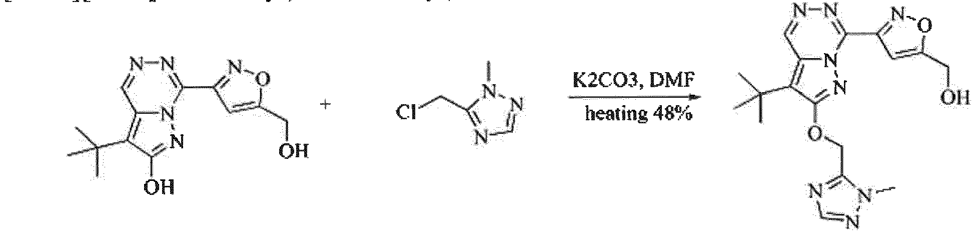

3-tert-butyl-7-(5-(hydroxymethyl)isoxazol-3-yl)pyrazolo[1,5-d][1,2,4] triazin-2-ol (360 mg, 1.2 mmol) was dissolved in DMF (10 mL), and 5-(chloromethyl)-1-methyl-1H-1,2,4- triazole hydrochloride (250 mg, 1.5 mmol) was added. Then potassium carbonate (1 g, 7.5 mmol) was added. The reacting solution was heated for 5 hours at 70°C under the condition of argon. The solvent was evaporated and methylbenzene was added for azeotropy. The residue was distributed between EtOAc and water to separate the organic layer. The water layer was extracted by EtOAc (30 mL x 2). The organic mixture was washed by brine and desiccated by sodium sulfate. The solid product was obtained after evaporation. The residue was conducted for chromatography on silica gel. With DCM : MeOH = 50:1 for elution, 260 mg product was obtained, which was a yellow powder. 1H NMR (400 MHz, DMSO-d6) δ: 9.60 (s, 1H), 7.94 (s, 1H), 7.22 (s, 1H), 5.85 (m, 1H), 5.62 (s, 2H), 4.74 (s, 2H), 3.91(s, 3H), 1.45 (s, 9H), -0.02 (s, 6H); ESI-MS: m/z 385 [M+H]+.

The following compounds (according to the invention) could be synthesized by using the methods above:
Using 3-tert-butyl-7-(5-(hydroxymethyl)isoxazol-3-yl)pyrazolo[1,5-d][1,2,4] triazin-2-yl and 2-chloromethylpyridine hydrochloride and the methods similar with those above, the following compound could be synthesized: (C2)(3-(3-tert-butyl-2-(2-pyridylmethoxyl)-3,3a-2H-pyrazolo[1,5-d][1,2,4] triazin-7-yl) isoxazole-5-yl)methanol, as shown in the figure below: m/z 381 [M+H]+.

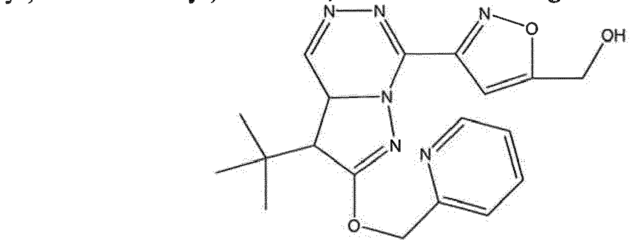

Similarly, following compounds could be synthesized: compound 3 (C3) (3-(3-tert-butyl-2-(3-pyridylmethoxyl)-3,3a-2H-pyrazolo[1,5-d] [1,2,4] triazin-7-yl) isoxazole-5-yl)methanol: m/z 381 [M+H]+.

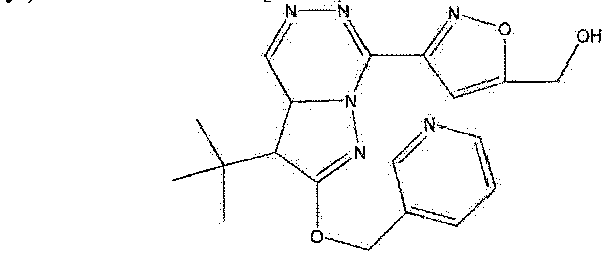

Compound 4 (C4) (3-(3-tert-butyl-2-(4-pyridylmethoxyl)-3,3a-2H-pyrazolo[1,5-d][1,2,4] triazin-7-yl) isoxazole-5-yl)methanol: m/z 381 [M+H]+.

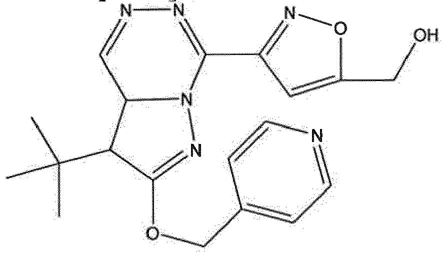

### Example 9. Compound C2, C3 and C4 suppressed neurogenic pain and inflammatory pain (according to the invention)

Compound C2, C3 and C4 were dissolved in 70% (PGE400:water=70:30 by volume) PGE400, respectively. The doses were 3 mg/kg, respectively, for injection.

Here, the inventors detected the analgesic effect of compound C2, C3 and C4 (Fig. 7A) in SNI model and inflammatory pain model.

Fig. 7A shows the chemical structural formula of compound C2, C3 and C4.

Fig. 7B indicated that intraperitoneal injection of compound C2, C3 and C4 efficiently treated neuropathic pain. In this experiment, at 14 days after the establishment of SNI model, compound C2, C3 and C4 were subcutaneously injected (3 mg/kg body weight), respectively. The pain threshold of rats was measured at 1 hour after administration. The results showed that these compounds efficiently suppressed neuropathic pain.

In CFA induced inflammatory pain model, at 3 days or 7 days after the establishment of the model, compound C2, C3 and C4 were intraperitoneally injected (3 mg/kg body weight) by the inventors. Then the effect of drugs on heat pain or mechanical pain was detected 1 hour later. As shown in Fig. 7C and D, each of this series of compounds efficiently suppressed heat pain or mechanical pain response.

### Example 10. (reference) The preparation of {3-[6-(Pyridin-2-ylmethoxy)-[1,2,4]triazolo [3,4-a]phthalazin-3-yl]-isoxazol-5-yl}-methanol and its analogs

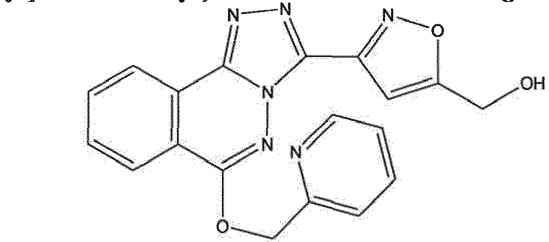

### 1. The preparation of intermediate 4: 1-(1-chlorophthalazin-4-yl) Intermediate 1: 1-(1-chlorophthalazin-4-yl)

### (a) 1,4-dichlorophthalazine

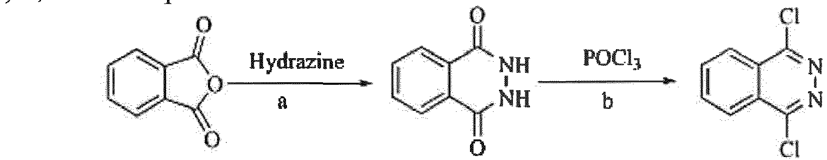

Isobenzofuran (5.92 g, 0.04 mol) was dissolved in acetic acid (22 mL) and hydrazine (2.8 mL, 0.044 mol) was added dropwise. The solution was refluxed for 4 hours and cooled to room temperature; then filtered and washed by ethanol. Then the solution was evaporated in vacuum and the product 2,3-dihydrophathalazine-1,4-dione (6.13 g, yield rate 94.6%) was obtained. The product was a white solid. The product was dissolved in POC13 (50 mL), mixing for 3 hours at 110°C. After cooling to room temperature, it was poured into cold water slowly. After filtration and washing by water, 1,4-dichlorophthalazine, a white solid, was obtained through recrystallization under the condition of THF.

### (b) 1-(1-chlorophthalazin-4-yl)hydrazine

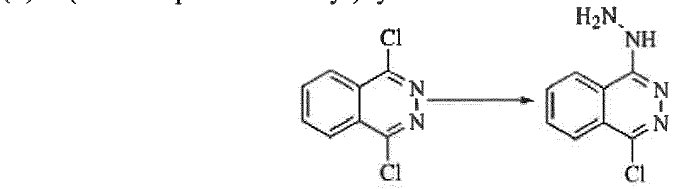

Hydrazine monohydrate (5.6 mL, 0.17 mol) was boiled in ethanol (150 mL), and then 1,4-dichlorophthalazine (3.0 g, 0.015 mol) was added, refluxing for 0.5 hour. The mixture was cooled to room temperature and filtered to collect solid that was then washed by ether. Recrystallization was conducted the condition of petroleum ether/ethyl acetate. The product (1-(1-chlorophthalazin-4-yl)hydrazine) was obtained, which was a faint yellow solid.

### Intermediate 2: 5-(hydroxymethy)isozazole-3-carboxylic acid

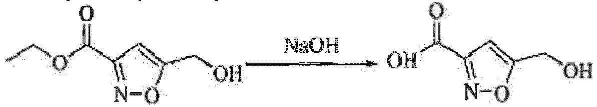

(a) For the methods of obtaining ethyl 5-(hydroxymethyl) wasoxazole-3-carboxylate, please refer to Example 8. Ethyl 5-(hydroxymethyl) wasoxazole-3-carboxylate (see Example 8, 1.5 g; 8.76 mmol) and 1 M sodium hydroxide (18 mL, 18 mmol) were mixed, stirring for 1.5 hours at room temperature. Brine (40 mL) was added and 6N hydrochloric acid was used to adjust the pH value to 2. Acetic ether (6X 60 mL) was used for extraction and then magnesium sulphate was used for desiccation. After evaporation, the product 5-(hydroxymethy)isozazole-3-carboxylic acid (1.2 g, yield rate 95%) was obtained, which was a white solid.

### Step 1:

### (a) N'-(1-chlorophthalzin-4-yl)-5-(hydroxymethy)isoxazole-3-carbohydrazide

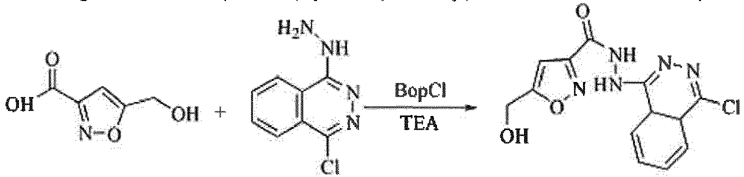

5-(hydroxymethy)isozazole-3-carboxylic acid (770 mg, 5.4 mmol) and triethylanmine (1.1 g, 10.8 mmol, 2 eq.) were dissolved in dry CH₂Cl₂ (100 mL). Bis(2-oxo-3-oxazolidiny)phosphonic chloride (BOP-C1, 1.47 g, 5.4 mmol, 1 eq.) was added at 0°C, stirring for 0.5 hour at 0°C. Then 1-(1-chlorophthalazin-4-yl)hydrazine (943 mg, 4.86 mmol, 0.9 eq.) was added, stirring for 2 hours at 0°C. Then the mixture was stirred overnight at room temperature. After evaporation, the solid residue was washed by water and ether, and the product N'-(1-chlorophthalzin-4-yl)-5-(hydroxymethy)isoxazole- 3-carbohydrazide was obtained (900 mg, yield rate 60%), which was a yellow solid.

### (b) (3-(6-chloro-[1,2,4]triazolo[3,4-a]pthalazin-3-yl)isoxazol-5-yl)methanol

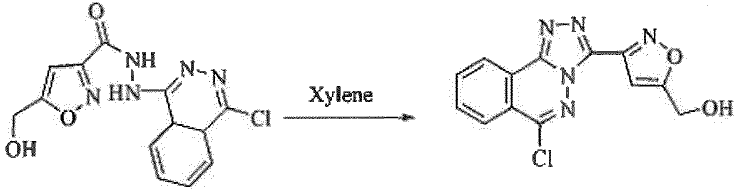

N'-(1-chlorophthalzin-4-yl)-5-(hydroxymethy)isoxazole-3-carboxylic ethyl ester (900 mg, 2.82 mmol) and ethylamine quinidine (154 mg, 0.13 mmol, 0.4 eq.) were refluxed in xylene (60 mL) for 3 hours. DCM (CH₂Cl₂) was added into the suspension to form uniform mixture. The organic layer was washed by water and desiccated by magnesium sulphate. After filtration and evaporation, the product (3-(6-chloro-[1,2,4]triazolo[3,4-a]phthalazin-3-yl) isoxazol-5-yl)methanol (300 mg, yield rate 35%) was obtained, which was a yellow solid.

### (c) {3-[6-(Pyridin-2-ylmethoxy)-[1,2,4]triazolo[3,4-a]phthalazin-3-yl]-isoxazol-5-yl}-met hanol

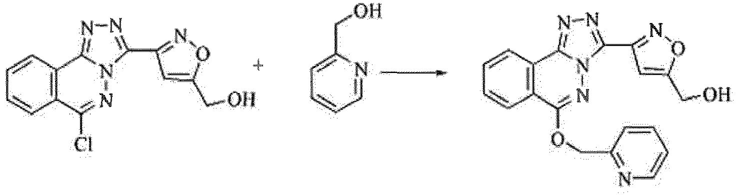

Pyridin-2-yl-methanol (300 mg, 1 mmol) was dissolved in DMF (30 mL), and NaH (80 mg, 2 mmol, 2eq.) was added at room temperature. The mixture was stirred for 15 minutes at room temperature. (3-(6-chloro-[1,2,4]triazolo[3,4-a]phthalazin-3-yl)isoxazol-5-yl)methanol (164 mg, 1.5 mmol, 1.5 eq.) was added, stirring for 1 hour at room temperature. Cold water was used to terminate the reaction and concentration was carried out. The residue was conducted for chromatography on silica gel and product C5 (50 mg, yield: 13.4%) was obtained. ¹HNMR (400 MHz, DMSO-d₆) δ:8.64(d,1H),8.63-8.60(d,1H), 8.53-8.33(d,1H), 8.13(t,1H) 7.99 (t, 1H),7.87 (t,1H),7.76(d,1H), 7.41-7.39(t,1H), 7.15(s,1H),5.87(t,1H),5.71 (s,2H),4.74-4.73(d,2H). m/z(ESI+)375(M+H⁺).

The following compounds could be synthesized by using the methods above:

Compound 6 (C6): {3-[6-(Pyridin-3-ylmethoxy)-[1,2,4]triazolo[3, 4-a]phthalazin-3-yl]-isoxazol-5-yl}-methanol, m/z(ESI+)375(M+H⁺).

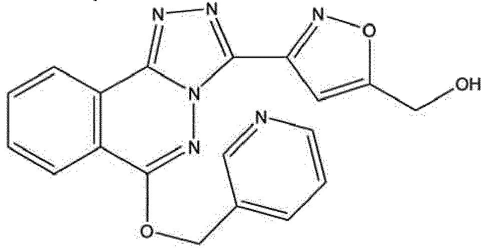

Compound 7 (C7): {3-[6-(Pyridin-4-ylmethoxy)-[1,2,4]triazolo[3, 4-a]pthalazin-3-yl-isoxazol-5-yl}-methanol, m/z(ESI+)375(M+H⁺).

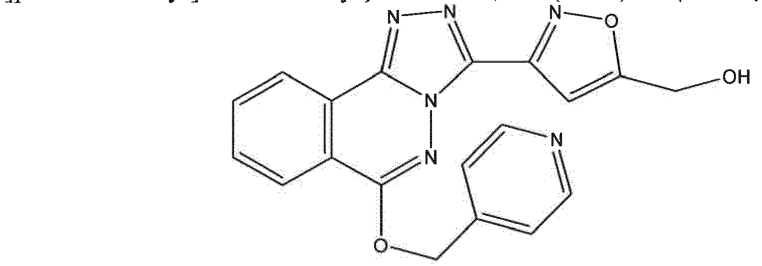

Compound 8 (C8): {3-[6-(2-bromo-phenyl)-[1,2,4]triazolo[3, 4-a]phthalazin-3-yl]-isoxazol-5-yl}-methanol, ¹HNMR (400 MHz, DMSO-d6) δ : 8.60(d,1H),8.27(d, 1H), 8.13(t, 1H),7.99(d, 1H), 7.85(d, 1H), 7.50(t, 1H), 7.47(t, 1H), 7.41(t, 1H), 7.21(s, 1H),5.85(t, 1H),5.71(s, 2H),4.75(d, 2H);4.75(d, 2H).

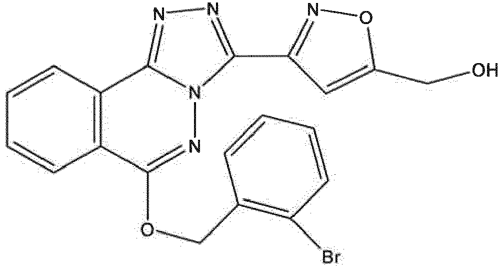

### Example 11. Compound C5, C6, C7 and C8 suppressed neurogenic pain and inflammatory pain (reference)

Compound C5, C6, C7 and C8 synthesized in the examples above were dissolved in 70% PGE400, respectively. The doses were 3 mg/kg, respectively, for injection.

Here, the inventors detected the analgesic effect of compound C5, C6, C7 and C8 (Fig. 8A) in SNI model and inflammatory pain model.

Fig. 8A shows the chemical structural formula of compound C5, C6, C7 and C8.

Fig. 8B indicated that intraperitoneal injection of compound C5, C6, C7 and C8 efficiently treated neuropathic pain. In this experiment, at 14 days after the establishment of SNI model, compound C5, C6, C7 and C8 were subcutaneously injected (3 mg/kg body weight), respectively. The pain threshold of rats was measured at 1 hour after administration. The results showed that these compounds efficiently suppressed neuropathic pain.

In CFA induced inflammatory pain model, at 3 days or 7 days after the establishment of the model, compound C5, C6, C7 and C8 were intraperitoneally injected (3 mg/kg body weight) by the inventors. Then the effect of drugs on heat pain or mechanical pain was detected 1 hour later. As shown in Fig. 8C and D, each of the series of compounds efficiently suppressed heat pain or mechanical pain response.

In behavior detection, the analgesic effect of C5 was significant. In Fig. 8E, C5 was subcutaneously injected at the doses of 1 mg/kg body weight and 3 mg/kg body weight, respectively. Then the pain threshold of rats was measured at 0.5 hour, 1 hour, 2 hours, 3 hours, 6 hours and 12 hours after administration. The results indicated that this compound efficiently suppressed neuropathic pain. In this experiment, morphine was used as positive control. It was subcutaneously injected at the dose of 10 mg/kg. Meanwhile, the inventors detected whether C5 affected the central response of brain. We detected whether C5 would potentially induce epileptic response. In Fig. 8F, it was found that there was no effect of C5 on pentetrazol induced epilepsy at the doses of 1 mg/kg, 3 mg/kg, 10 mg/kg and 15 mg/kg, suggesting that C5 was capable of efficiently treating pain with no potential central side effect.

### Example 12. Verification of the analgesic effect of a sort of inverse agonists of α5-GABA_{A} receptor (reference)

The inverse agonists of α5-GABA_{A} receptor are widely used for treating cognition-related diseases. F. Hoffmann-La Roche Co. Ltd. developed a series of related compounds and reported 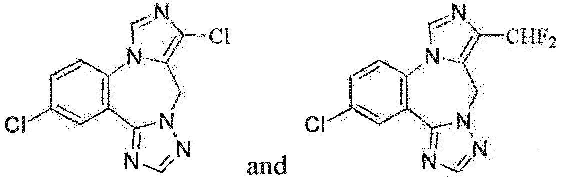 as potential drugs in literature for Phase I clinical trial. Here, according to literature (Knust H et al., The discovery and unique pharmacological profile of RO4938581 and RO4882224 as potent and selective GABA_{A} alpha5 inverse agonists for the treatment of cognitive dysfunction. Bioorg Med Chem letter. 2009 Oct 15;19(20):5940-4) and US patents (US 2006/0084642 A1, US 2007/0082890 A1, etc.), the inventors synthesized the compounds shown in the table below. In rats chronic pain model, the effect of this series of compounds on pain was detected. As shown in the figure below, these compounds were dissolved in PGE400 (PGE400: water=30: 70 by volume), respectively. The doses were 3 mg/kg, respectively, for injection.

The analgesic effect in SNI model and inflammatory model. Behavioral data indicated that intraperitoneal injection of the following compounds (3 mg/kg body weight) efficiently treated neuropathic pain. In this experiment, at 14 days after SNI model, the following compounds were subcutaneously injected (3 mg/kg body weight). The pain threshold of rats was measured at 1 hour after administration. The results showed that these compounds efficiently suppressed neuropathic pain. In CFA-induced inflammatory pain model, at 3 days or 7 days after CFA injection, the following compounds were intraperitoneally injected (3 mg/kg body weight). Then the analgesic effect of drugs on heat pain or mechanical pain was detected 1 hour later. The results showed that the series of compounds could efficiently suppressed heat pain or mechanical pain response.

Meanwhile, the inventors detected the distribution of these compounds in the tissue of rats. Firstly the substances to be measured were subcutaneously injected into the body of animals. Then brain tissue and plasma tissue were extracted 1 hour after injection. Ls-MS was used to measure the concentration of drugs. If the brain/plasma ratio of the compound is less than 0.1, then the candidate substance is a potential drug for treating pain with fewer side effects. The inventors found that 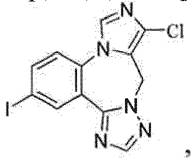 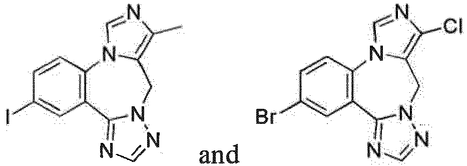 barely passed blood brain barrier.

**Table 3**

| Compound | Brain/plasma ratio | CFA induced inflammatory pain model (heat pain) | CFA induced inflammatory pain model (mechanical pain) | Neuropathic pain (SNI model) |
|---|---|---|---|---|
| 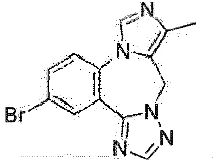 | <0.2 | Significant analgesic effect | Significant analgesic effect | Significant analgesic effect |
| 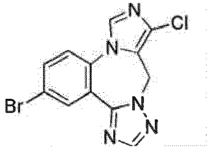 | <0.1 | Significant analgesic effect | Significant analgesic effect | Significant analgesic effect |
| 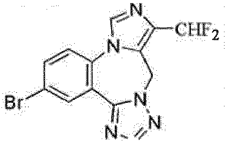 | >0.2 | Significant analgesic effect | Significant analgesic effect | Significant analgesic effect |
| 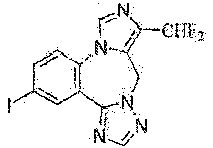 | NA | Significant analgesic effect | Significant analgesic effect | Significant analgesic effect |
| 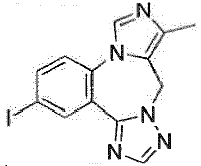 | <0.1 | Significant analgesic effect | Significant analgesic effect | Significant analgesic effect |
| 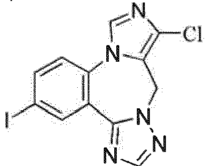 | <0.1 | Significant analgesic effect | Significant analgesic effect | Significant analgesic effect |
| 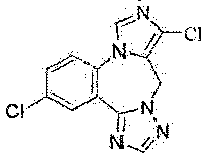 | >0.2 | Significant analgesic effect | Significant analgesic effect | Significant analgesic effect |
| 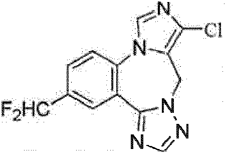 | NA | Significant analgesic effect | Significant analgesic effect | Significant analgesic effect |
| 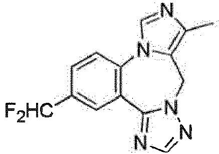 | NA | Significant analgesic effect | Significant analgesic effect | Significant analgesic effect |
| 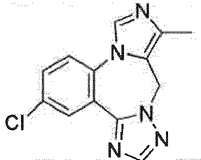 | =0.2 | Significant analgesic effect | Significant analgesic effect | Significant analgesic effect |

Each of the compounds in Table 3 has significant analgesic effect, wherein compound 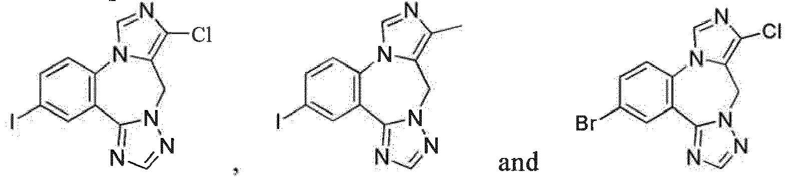 can efficiently suppress CFA induced inflammatory pain as well as SNI induced neuropathic pain. These three compounds can barely pass blood brain barrier, and therefore will not affect central function. These compounds are the drugs that can effectively treat pain with fewer central side effects.

## Claims

1. An inverse agonist of γ-aminobutyric acid A receptor that contains α5 subunit and its pharmaceutically acceptable salt for use in preventing, relieving or treating pain, wherein said inverse agonist of γ-aminobutyric acid A receptor that contains α5 subunit is a compound or its pharmaceutically acceptable salt selected from:

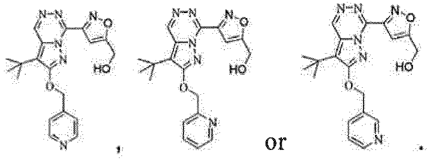

2. The inverse agonist for use according to claim 1, wherein the inverse agonist of γ-aminobutyric acid A receptor that contains α5 subunit can bind to the γ-aminobutyric acid A receptor that contains α5 subunit expressed in peripheral nerve, but cannot bind to the γ-aminobutyric acid A receptor that contains α5 subunit expressed in central nervous system.

3. The inverse agonist for use according to claim 1, wherein the pain is peripheral nerve associated chronic pain.

4. The inverse agonist for use according to claim 3, wherein the chronic pain is neuropathic pain, inflammatory pain or cancer pain.

5. The inverse agonist for use according to claim 1, wherein the pain is selected from the group consisting of: headache, facial pain, neck pain, shoulder pain, back pain, thoracic pain, abdominal pain, dorsopathy, waist pain, lower limb pain, muscle and bone pain, body pain, vascular pain, gout associated pain, arthritis pain, somatoform disorder associated pain, visceral pain, the pain caused by infectious diseases, boniness pain, sickle cell anemia associated pain, autoimmune disease associated pain, multiple sclerosis or inflammation associated pain, acute or chronic inflammatory pain, cancer pain, neuropathic pain, injury or surgery caused pain, nociceptive pain, diabetes associated pain, peripheral neuropathies, postherpetic neuralgia, trigeminal neuralgia, waist or cervix radiculopathy, glossopharyngeal neuralgia, autonomic nerve reflex pain, reflex sympathetic dystrophy associated pain, nerve root avulsion associated pain, chemical injury associated pain, toxin associated pain, nutrition deficiency associated pain, virus or bacteria infection associated pain, degenerative osteoarthropathy or the combination thereof.

## Patentansprüche

1. Inverser Agonist von γ-Aminobuttersäure-A-Rezeptor, der eine α5-Untereinheit enthält, und sein pharmazeutisch unbedenkliches Salz zur Verwendung beim Vorbeugen, Stillen oder Behandeln von Schmerz, wobei der inverse Agonist von γ-Aminobuttersäure-A-Rezeptor, der eine α5-Untereinheit enthält, eine Verbindung oder ihr pharmazeutisch unbedenkliches Salz ist, das ausgewählt ist aus:

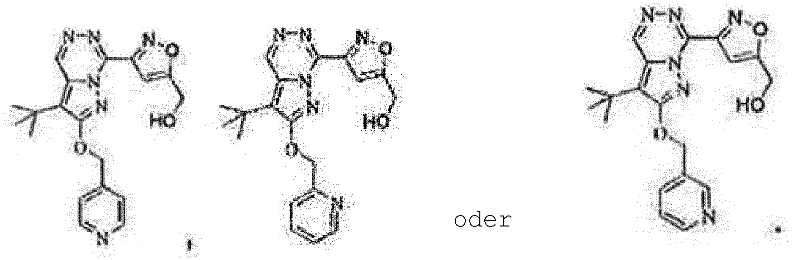

2. Inverser Agonist zur Verwendung nach Anspruch 1, wobei sich der inverse Agonist von γ-Aminobuttersäure-A-Rezeptor, der eine α5-Untereinheit enthält, an den γ-Aminobuttersäure-A-Rezeptor binden kann, der eine α5-Untereinheit enthält, die in einem peripheren Nerv exprimiert ist, aber sich nicht an den γ-Aminobuttersäure-A-Rezeptor binden kann, der eine α5-Untereinheit enthält, die im Zentralnervensystem exprimiert ist.

3. Inverser Agonist zur Verwendung nach Anspruch 1, wobei der Schmerz ein mit einem peripheren Nerv verbundener chronischer Schmerz ist.

4. Inverser Agonist zur Verwendung nach Anspruch 3, wobei der chronische Schmerz ein neuropathischer Schmerz, ein Entzündungsschmerz oder ein Krebsschmerz ist.

5. Inverser Agonist zur Verwendung nach Anspruch 1, wobei der Schmerz aus folgender Gruppe ausgewählt ist: Kopfschmerz, Gesichtsschmerz, Nackenschmerz, Schulterschmerz, Rückenschmerz, Brustschmerz, Bauchschmerz, Dorsopathie, Hüftschmerz, Schmerzen der unteren Gliedmaßen, Muskel- und Knochenschmerz, Körperschmerz, Gefäßschmerz, Gicht-bezogener Schmerz, Arthritisschmerz, Schmerz in Verbindung mit somatoformer Störung, Eingeweideschmerz, durch Infektionskrankheiten verursachter Schmerz, Knochigkeitsschmerz, Sichelzellenanämie-bezogener Schmerz, Schmerz in Verbindung mit Autoimmunkrankheiten, Schmerz in Verbindung mit multipler Sklerose oder Entzündungen, akuter oder chronischer Entzündungsschmerz, Krebsschmerz, neuropathischer Schmerz, Schmerz in Verbindung mit Verletzungen oder chirurgischen Eingriffen, Nozizeptorenschmerz, Diabetes-bezogener Schmerz, periphere Neuropathien, Post-Zoster-Neuralgie, Trigeminusneuralgie, Hüft- oder zervikale Radikulopathie, Glossopharyngeusneuralgie, Schmerz in Verbindung mit autonomen Nervenreflexen, Schmerz in Verbindung mit reflexsympathetischer Dystrophie, Schmerz in Verbindung mit Nervenwurzelavulsion, Schmerz in Verbindung mit chemischen Verletzungen, Toxin-bezogener Schmerz, Ernährungsmangel-bezogener Schmerz, Virus- oder Bakterieninfektions-bezogener Schmerz, degenerative Osteoarthropathie oder Kombinationen davon.

## Revendications

1. Agoniste inverse du récepteur A de l'acide γ-aminobutyrique qui contient la sous-unité α5 et son sel pharmaceutiquement acceptable, pour une utilisation dans la prévention, le soulagement ou le traitement de la douleur, ledit agoniste inverse du récepteur A de l'acide γ-aminobutyrique qui contient la sous-unité α5 étant un composé, ou son sel pharmaceutiquement acceptable, choisi parmi :

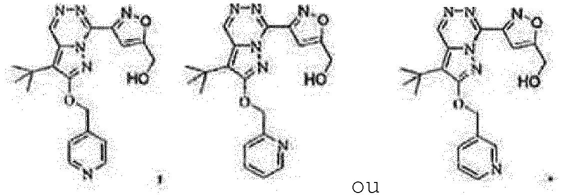

2. Agoniste inverse pour l'utilisation selon la revendication 1, l'agoniste inverse du récepteur A de l'acide γ-aminobutyrique qui contient la sous-unité α5 pouvant se lier au récepteur A de l'acide γ-aminobutyrique qui contient la sous-unité α5 exprimé dans le nerf périphérique, mais ne pouvant se lier au récepteur A de l'acide γ-aminobutyrique qui contient la sous-unité α5 exprimé dans le système nerveux central.

3. Agoniste inverse pour l'utilisation selon la revendication 1, pour lequel la douleur est une douleur chronique associée au nerf périphérique.

4. Agoniste inverse pour l'utilisation selon la revendication 3, pour lequel la douleur chronique est une douleur neuropathique, une douleur inflammatoire ou une douleur cancéreuse.

5. Agoniste inverse pour l'utilisation selon la revendication 1, pour lequel la douleur est choisie dans le groupe consistant en la céphalalgie, la douleur faciale, la douleur du cou, la douleur des épaules, la douleur du dos, la douleur thoracique, la douleur abdominale, la dorsopathie, la douleur lombaire, la douleur des membres inférieurs, la douleur des muscles et des os, la douleur corporelle, la douleur vasculaire, la douleur associée à la goutte, la douleur arthritique, la douleur associée au trouble somatoforme, la douleur viscérale, la douleur provoquée par des maladies infectieuses, la douleur osseuse, la douleur associée à la drépanocytose, la douleur associée aux maladies auto-immunes, la douleur associée à la sclérose en plaques ou à une inflammation, la douleur inflammatoire aiguë ou chronique, la douleur cancéreuse, la douleur neuropathique, la douleur provoquée par une lésion ou une intervention chirurgicale, la douleur nociceptive, la douleur associée au diabète, les neuropathies périphériques, l'algie post-zona, la névralgie trigéminale, la radiculopathie lombaire ou cervicale, la névralgie glossopharyngienne, la douleur réflexe du système nerveux autonome, la douleur associée à une dystrophie sympathique réflexe, la douleur associée à l'extraction des racines nerveuses, la douleur associée à une lésion chimique, la douleur associée à une toxine, la douleur associée à une carence alimentaire, la douleur associée à une infection virale ou bactérienne, une ostéo-arthropathie dégénérative, ou une combinaison de celles-ci.


### SEQUENCE LISTING

<110> SHANGHAI INSTITUTES FOR BIOLOGICAL SCIENCES, CHINESE ACADEMY OF SCIENCES; Shanghai Institute of Materia Medica Chinese Academy of SCIENCES
<120> Substances for treatment or relief of pain
<130> 106305 1PCEP
<160> 2
<170> PatentIn version 3.3
<210> 1
   <211> 21
   <212> DNA
   <213> artificial sequence
<220>
   <223> siRNA
<220>
   <221> misc_feature
   <223> siRNA
<400> 1
   ggugcgaaca gacaucuaut t 21
<210> 2
   <211> 21
   <212> DNA
   <213> artificial sequence
<220>
   <223> siRNA
<220>
   <221> misc_feature
   <223> siRNA
<400> 2
   uucuccgaac gugucacgut t 21